(19) Europäisches Patentamt
European Patent Office
Office européen des brevets

(11) EP 3 643 317 B1

(12) **EUROPEAN PATENT SPECIFICATION**

(45) Date of publication and mention of the grant of the patent:
**09.07.2025 Bulletin 2025/28**

(21) Application number: **18821566.9**

(22) Date of filing: **20.06.2018**

(51) International Patent Classification (IPC):
***A61K 35/545*** *(2015.01)* ***A61P 25/14*** *(2006.01)*
***A61P 25/28*** *(2006.01)* ***C12N 5/0775*** *(2010.01)*

(52) Cooperative Patent Classification (CPC):
**A61P 25/14; A61K 35/545; A61P 25/28;**
A01K 2207/30; A01K 2227/105; A01K 2267/035

(86) International application number:
**PCT/JP2018/023511**

(87) International publication number:
**WO 2018/235878 (27.12.2018 Gazette 2018/52)**

(54) **AMELIORATION AND TREATMENT OF BRAIN DISORDER RESULTING FROM FETAL GROWTH RETARDATION USING PLURIPOTENT STEM CELLS**

BESSERUNG UND BEHANDLUNG VON HIRNSTÖRUNGEN, DIE AUS EINER FETALEN WACHSTUMSVERZÖGERUNG RESULTIEREN, UNTER VERWENDUNG PLURIPOTENTER STAMMZELLEN

AMÉLIORATION ET TRAITEMENT D'UN TROUBLE CÉRÉBRAL RÉSULTANT D'UN RETARD DE CROISSANCE DE FOETUS À L'AIDE DE CELLULES SOUCHES PLURIPOTENTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2017 JP 2017120900**

(43) Date of publication of application:
**29.04.2020 Bulletin 2020/18**

(73) Proprietors:
- **National University Corporation Tokai National Higher Education and Research System**
  **Nagoya-shi, Aichi 464-8601 (JP)**
- **Tohoku University**
  **Sendai-shi, Miyagi 980-8577 (JP)**
- **National Cerebral and Cardiovascular Center**
  **Suita-shi, Osaka 564-8565 (JP)**
- **Life Science Institute, Inc.**
  **Tokyo 100-8251 (JP)**

(72) Inventors:
- **SATO, Yoshiaki**
  **Nagoya-shi**
  **Aichi 464-8601 (JP)**
- **KITASE, Yuma**
  **Nagoya-shi**
  **Aichi 464-8601 (JP)**
- **SHIMIZU, Shinobu**
  **Nagoya-shi**
  **Aichi 464-8601 (JP)**
- **MIZUNO, Masaaki**
  **Nagoya-shi**
  **Aichi 464-8601 (JP)**
- **HAYAKAWA, Masahiro**
  **Nagoya-shi**
  **Aichi 464-8601 (JP)**
- **DEZAWA, Mari**
  **Sendai-shi**
  **Miyagi 980-8577 (JP)**
- **TSUJI, Masahiro**
  **Suita-shi**
  **Osaka 565-8565 (JP)**

(74) Representative: **AWA Sweden AB**
**Junkersgatan 1**
**582 35 Linköping (SE)**

(56) References cited:
**EP-A1- 3 459 553** **EP-A1- 3 643 317**
**WO-A1-2017/199976** **US-A1- 2016 058 800**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 643 317 B1

- DATABASE WPI Week 201851, Derwent World Patents Index; AN 2018-56696N, XP002801813
- SATO, YOSHIRO: "Development of novel treatment method for perinatal disease using muse cell", PROGRAMS AND ABSTRACTS OF THE 16TH REGENERATIVE MEDICINE GENERAL CONFERENCE OF THE JAPANESE SOCIETY FOR REGENERATIVE MEDICINE, vol. 16, 1 February 2017 (2017-02-01), pages 179, XP009513414, ISSN: 1347-7919
- SUZUKI, TOSHIHIKO ET AL.: "Stem cell therapy for hypoxic-ischemic encephalopathy in infants using multilineage-differentiating stress enduring cells", JOURNAL OF JAPAN SOCIETY OF PERINATAL AND NEONATAL MEDICINE, vol. 52, no. 2, June 2016 (2016-06-01), pages 608, XP009518554, ISSN: 1348-964X
- HATTORI, TETSUO ET AL.: "Special edition: several problems about SGA - Complications of SGA infants during NICU hospitalization and management central nervous system", PERINATAL MEDICINE, vol. 40, no. 2, 2010, pages 205 - 208, XP009518553, ISSN: 0386-9881


Remarks:

The file contains technical information submitted after the application was filed and not included in this specification

## Description

FIELD

The present invention relates to a cell preparation for regenerative medicine. More specifically, it relates to an effective cell preparation for treatment of brain disorder associated with fetal growth retardation, which contains pluripotent stem cells, and to a novel treatment method.

BACKGROUND

Fetal growth retardation (FGR, or intrauterine growth restriction: IUGR) occurs due to a variety of causes, but FGR resulting from middle pregnancy-onset pregnancy hypertension syndrome is one type in which chronic hypoxemia, circulatory failure and malnutrition through the placenta act in combination as a cause of post-neurological disorders including mental development delay and cognitive impairment, and it has no established method of treatment. Fetal growth retardation is currently seen in approximately 8 to 10% of all pregnancies, with a perinatal death rate of about 18% and a fetal death rate of about 31%. Fetal growth retardation is also a cause of a condition in which the body height and body weight at birth are both below standard values (small-for-gestational age: SGA), and it constitutes a high-risk group for post-neurological disorder. While various therapeutic interventions have been attempted to prevent such post-neurological disorders, none have been completely effective (NPL 1).

Recently in the field of regenerative medicine, research has been conducted on cell therapy using stem cells for a variety of diseases, and embryonic stem cells (ES cells), nerve stem/progenitor cells (NSPC), induced pluripotent stem cells (iPS cells) and umbilical cord blood stem cells (UCBC) have become known as types of stem cells that are expected to have potential in clinical applications.

The bone marrow mesenchymal cell fraction (MSC) has been isolated from adults, and it is known to have the ability to differentiate into bone, cartilage, adipocytes, neurons and skeletal muscle, for example (NPLs 2 and 3). However, MSCs are a cell group including various types of cells, the nature of whose differentiation potency is unknown, and they have wide variation in therapeutic effect. iPS cells (PTL 1) have been reported as adult pluripotent stem cells, but establishing iPS cells requires the very complex procedure of introducing specific genes or specific compounds into the skin fibroblast fraction (mesenchymal cell fraction) of somatic cells, while iPS cells also have high tumor-forming potential, and therefore very high hurdles stand in the way of their clinical application.

Research by Prof. Desawa, one of the present inventors, has demonstrated that the pluripotency of the mesenchymal cell fraction is exhibited by pluripotent stem cells that express SSEA-3 (Stage-Specific Embryonic Antigen-3) as a surface antigen (Multilineage-differentiating Stress Enduring cells, or Muse cells), which are present in the mesenchymal cell fraction and can be obtained without inducing, and that this holds potential for application in treatment of diseases by tissue regeneration. It has also been found that Muse cells can be concentrated by stimulation of the mesenchymal cell fraction via different kinds of stress (PTL 2, NPL 4). However, it has not yet been demonstrated that the expected therapeutic effect can be obtained using Muse cells for amelioration and/or treatment of brain disorders associated with fetal growth retardation.

[CITATION LIST]

[PATENT LITERATURE]

[PTL 1] Japanese Patent Publication No. 4183742
[PTL 2] International Patent Publication No. WO2011/007900
[PTL 3] International Patent Publication No. WO2017/199976

[NON PATENT LITERATURE]

[NPL 1] O'Keeffe M.J., et al., Pediatrics, Vol.112, p.301-307(2003)
[NPL 2] Dezawa, M., et al., J. Clin. Invest., Vol.113, p.1701-1710(2004)
[NPL 3] Dezawa, M., et al., Science, Vol.309, p.314-317(2005)
[NPL 4] Wakao, S, et al., Proc. Natl. Acad. Sci. USA, Vol.108, p.9875-9880(2011)

SUMMARY

[TECHNICAL PROBLEM]

It is an object of the present invention to provide a novel medical use for pluripotent stem cells (Muse cells) in regenerative medicine. More specifically, it is an object of the invention to provide a cell preparation and pharmaceutical composition that include Muse cells and that are effective for treating brain disorders associated with fetal growth retardation, as well as a novel treatment method. The invention is defined by the appended independent claim. Embodiments are set forth in the appended dependent claims and in the following description and drawings.

[SOLUTION TO PROBLEM]

The present inventors fitted an ameroid constrictor (AC) onto the uterine artery of pregnant rats to create a chronic ischemia model and administered Muse cells by intravenous injection, obtaining the finding that this ameliorated brain disorders associated with growth retardation (for example, learning disability and motor disability), and we have thereupon completed this invention.

Specifically, the present invention provides the following.

A cell preparation for use in amelioration and/or treatment of a brain disorder associated with fetal growth retardation due to hypoxemia caused by placental or umbilical cord factors, or due to complex and chronic factors such as circulatory failure and malnutrition, comprising an effective amount of pluripotent stem cells positive for SSEA-3 isolated from mesenchymal tissue or cultured mesenchymal cells of a living organism,wherein the brain disorder associated with fetal growth retardation is selected from the group consisting of abnormal quality of movement, abnormal neurological development, cerebral palsy, cognitive impairment, and behavior disorder.

[ADVANTAGEOUS EFFECTS OF INVENTION]

The present invention can drastically minimize brain disorders in subjects suffering from brain disorders associated with fetal growth retardation, by a brain tissue-regenerating mechanism in which Muse cells are administered through a vein or by another route to selectively accumulate them in damaged brain tissue, and the Muse cells differentiate to brain tissue-forming cells within the tissue.

BRIEF DESCRIPTION OF DRAWINGS

FIG. 1A shows the results of a negative geotaxis test for behavior improvement in a fetal growth retardation rat model, using a Muse cell-administered group ("Muse"), a non-Muse cell-administered group ("non-Muse"), a group administered a cell preservation solution (STEM-CELLBANKER$^R$) alone ("vehicle"), and a sham group. The results are shown for each group after testing once per day for 4 successive days, using 8-11 day-old rats.

FIG. 1B shows results of a negative geotaxis test, similar to FIG. 1A, but as the average value for each rat calculated as the total measured time (sec) during the 4 days divided by 4.

FIG. 2 shows the results of a Rota Rod test for motor function improvement in a fetal growth retardation rat model, using a Muse cell-administered group ("Muse"), a non-Muse cell-administered group ("non-Muse"), and a group administered a cell preservation solution (STEM-CELLBANKER$^R$) alone ("vehicle").

FIG. 3 shows the results of an open field test for emotional behavior (hyperactivity, etc.) in a fetal growth retardation rat model, using a Muse cell-administered group ("Muse"), a non-Muse cell-administered group ("non-Muse"), and a group administered a cell preservation solution (STEM-CELLBANKER$^R$) alone ("vehicle"). A shows the results for traveling distance of the test animal for a prescribed time period, B shows the results for time immobile, and C shows the results for mobile episodes.

FIG. 4A shows the results of a negative geotaxis test for behavior improvement in a fetal growth retardation rat model, using a Muse cell-administered group ("Muse"), a non-Muse cell-administered group ("non-Muse"), a group administered a cell preservation solution (STEM-CELLBANKER$^R$) alone ("vehicle"), and a sham group. A shows the results for each group in a geotaxis test using 8 to 11 day-old rats, the test being conducted once per day for 4 successive days.

In FIG. 4B, B is the average value for each rat, calculated as the total measured time (sec) during the 4 days divided by 4, similar to FIG. 1A.

FIG. 5 shows the results of a negative geotaxis test for behavior improvement in a fetal growth retardation rat model, using a Muse cell-administered group ("Muse"), a non-Muse cell-administered group ("non-Muse"), a group administered a cell preservation solution (STEM-CELLBANKER$^R$) alone ("vehicle"), and a sham group.

FIG. 6 shows the results of a novel object recognition test, for improvement in memory learning and visual cognitive memory in a fetal growth retardation rat model, using a Muse cell-administered group ("Muse"), a non-Muse cell-administered group ("non-Muse"), a group administered a cell preservation solution (STEM-CELLBANKER$^R$) alone ("vehicle"), and a sham group. The evaluation was conducted using the recognition index by the rats for a novel object.

FIG. 7 shows the results of a negative geotaxis test for behavior improvement in a fetal growth retardation rat model (9 days old), using a Muse cell-administered group ("Muse") (n = 21), a non-Muse cell-administered group ("non-Muse") (n = 20), a group with a base solution (HBSS solution) added alone ("vehicle") (n = 18) and a sham group (n = 25). The results are shown for each group in a geotaxis test using 8 to 11 day-old rats, the test being conducted once per day for 4 successive days.

FIG. 8 shows the results of a Rota Rod test for motor function improvement in a fetal growth retardation rat model (1 month after birth), using a Muse cell-administered group ("Muse") (n = 12), a non-Muse cell-administered group ("non-Muse") (n = 12), a group with a base solution (HBSS solution) added alone ("vehicle") (n = 9) and a sham group (n = 16).

FIG. 9 shows the results of a Y maze test for spontaneous movement activity and spatial working memory in a fetal growth retardation rat model (1 month after birth), using a Muse cell-administered group ("Muse") (n = 12), a non-Muse cell-administered group ("non-Muse") (n = 12), a group with a base solution (HBSS solution) added alone ("vehicle") (n = 11) and a sham group (n = 15).

FIG. 10 shows the results of a Rota Rod test for motor function improvement in a fetal growth retardation rat model (5 months after birth), using a Muse cell-administered group ("Muse") (n = 12), a non-Muse cell-administered group ("non-Muse") (n = 12), a group with a base solution (HBSS solution) added alone ("vehicle") (n = 11) and a sham group (n = 16).

FIG. 11 shows the results of an open field test for emotional behavior (hyperactivity, etc.) in a fetal growth retardation rat model (5 months after birth), using a Muse cell-administered group ("Muse") (n = 12), a non-Muse cell-administered group ("non-Muse") (n = 12), a group with a base solution (HBSS solution) added alone ("vehicle") (n = 11) and a sham group (n = 16). At left are shown the results for traveling distance of the test animal during a prescribed time period, at center are shown the results for time immobile, and at right are shown the number of mobile episodes (number of movements between light and dark regions).

FIG. 12 shows the results of a Rota Rod test for motor function improvement in a fetal growth retardation rat model (1 month after birth), using a Muse cell-administered group ("Muse") (n = 10), a non-Muse cell-administered group ("non-Muse") (n = 11), a group with a base solution (HBSS solution) added alone ("vehicle") (n = 9) and a sham group (n = 16).

FIG. 13 shows the results of a Rota Rod test for motor function improvement in a fetal growth retardation rat model (5 months after birth), using a Muse cell-administered group ("Muse") (n = 10), a non-Muse cell-administered group ("non-Muse") (n = 11), a group with a base solution (HBSS solution) added alone ("vehicle") (n = 9) and a sham group (n = 16).

## DESCRIPTION OF EMBODIMENTS

The present invention relates to a cell preparation and pharmaceutical composition for amelioration and/or treatment of brain disorders associated with fetal growth retardation, containing SSEA-3 positive pluripotent stem cells (Muse cells), as depicted in the appended claims. The present invention will now be explained in greater detail.

### 1. Applicable diseases and their diagnosis

The invention is directed toward amelioration and treatment of brain disorders associated with fetal growth retardation, using a cell preparation or pharmaceutical composition containing SSEA-3 positive pluripotent stem cells (Muse cells). Generally speaking, growth retardation in the perinatal period (for humans, this is the period from the 22nd week of pregnancy up until 7 days after birth) includes intrauterine (fetal) growth retardation occurring in the fetal stage, and extrauterine growth retardation in which postnatal physical growth is suppressed. The term "fetal growth retardation" (or "intrauterine growth retardation") refers to a condition in which fetal growth is inhibited due to some cause. Fetal factors include multiple conception, fetal malformation, chromosome abnormality and congenital infection (congenital rubella, congenital cytomegalovirus infection, congenital *Toxoplasma* infection, congenital herpes infection, etc.), and placental or umbilical cord factors include placental hypoplasia, subchorionic hematoma, placenta previa, chorioamnionitis, abnormal umbilical cord attachment, umbilical stem torsion and umbilical nodule. Maternal factors include underlying maternal diseases such as pregnancy hypertension syndrome, heart disease, hypertension, kidney disease, diabetes, collagen disease, respiratory disease and autoimmune disease, as well as lifestyle habit causes such as smoking, drinking and drug use. Intrauterine growth retardation fetuses are considered to have poor development prognosis compared to normal developing fetuses, even when excluding cases of fetal malformation, chromosomal aberration or congenital infection that clearly affect the fetal central nervous system. This tendency is known to be especially marked in cases of suppressed head circumference development.

Fetal growth retardation is largely classified as either "symmetrical" or "asymmetrical", depending on the period during which the fetus growth is inhibited. The "symmetrical" form is characterized by inhibited cell growth, creating a deficiency of cells, which suppresses the amount of growth of the neck, trunk and extremities and results in a proportional body frame but with an overall small size. Postnatal prognosis tends to be poor due to growth inhibiting factors in the fetus itself. The main causes attributed to the fetus during early gestation are chromosomal aberration, leading deformity, *in utero* infection and alcohol intoxication. The "asymmetrical" form, on the other hand, is characterized by small sizes of the cells in local regions (primarily the trunk), with development of the head but inhibited trunk growth, and therefore a lean body frame. The incidence frequency is about 2 to 3 times that of the "symmetrical" form. Postnatal prognosis is relatively good since the cause of growth inhibition is placental blood flow disturbance (fetal malnutrition).

The known definitions for "fetal growth retardation" used here include "the body height and body weight at birth are both below the 10th percentile of standard values" (SGA), "the estimated fetal body weight by development is below the 10th percentile for the number of weeks (or days) of pregnancy", and "no greater than -1.5 SD of the standard value when using a standardized fetal weight estimation formula". For diagnosis of fetal growth retardation, since there are no initial symptoms and judgment is usually made by ultrasonic examination, it is more advantageous to carry out diagnosis using a standardized fetal weight estimation formula (APEs method), as mentioned above. Specifically, the following formula is used.

$$EFW\ (g) = 1.07 \times BPD^3 + 0.30 \times AC^2 \times FL$$

(EFW: estimated fetal weight, (estimated fetal weight), BPD: biparietal diameter, AC: abdominal circumference, FL: femur length.

In a clinical setting, commonly a fetus diagnosed with fetal growth retardation is delivered during the appropriate period while judging the state of the fetus based on the Biophysical Profile Score (BPS), and it is then transferred for extrauterine treatment. Death, plethora, thrombocytopenia, leukocytopenia, cholestasis, gastrointestinal tract abnormalities, hypoglycemia, cardiac failure or pneumorrhagia, abnormal quality of movement or cerebral palsy may be observed at the postnatal and neonatal stages, low body height and abnormal neurological development (cognitive impairment, behavior disorder) may be observed at the infant stage, and impaired glucose tolerance, cardiac or vascular abnormalities, and abnormal lipid or blood sugar profile may be observed at the adult stage. Various complications are therefore associated with fetal growth retardation.

It is an object of the present invention to ameliorate and treat complications of fetal growth retardation, namely hypoxemia caused by placental or umbilical cord factors, or complications due to complex and chronic factors such as circulatory failure and malnutrition, and especially abnormal quality of movement (for example, reduced orientation and coordination of movement or reduced repertoire of movement) and abnormal neurological development (for example, reduced intelligence/-cognition, learning problems, behavioral problems or reduced societal adaptation). The present invention is thus targeted at amelioration and treatment of brain disorders associated with fetal growth retardation, but it is especially applicable for brain disorders caused by chronic hypoperfusion (ischemia).

According to the invention, the cell preparation and pharmaceutical composition described below are administered to (or "transplanted in", as it may also be termed hereunder) a subject for treatment of the applicable disease, to allow amelioration and/or treatment of the applicable disease. The term "amelioration" here means alleviating or suppressing brain disorders associated with fetal growth retardation, and its preferred meaning is alleviation of symptoms to an extent that is not a problem for daily life. The term "treatment" refers to suppressing or completely eliminating brain disorders associated with fetal growth retardation.

2. Cell preparation and pharmaceutical composition

(1) Pluripotent stem cells

The pluripotent stem cells to be used in the cell preparation and pharmaceutical composition of the present invention are typically cells whose existence in the human body was discovered by Prof. Dezawa, one of the present inventors, and which are named "Muse (Multilineage-differentiating Stress Enduring) cells". Muse cells can be obtained from bone marrow fluid and adipose tissue (Ogura, F., et al., Stem Cells Dev., Nov 20, 2013 (Epub) (published on Jan 17, 2014)) or from skin tissue such as dermal connective tissue, and they are widely dispersed throughout the connective tissue of various organs. The cells have the properties of both pluripotent stem cells and mesenchymal stem cells, and are identified as being double-positive for the cell surface markers "SSEA-3 (Stage-specific embryonic antigen-3)" and "CD105". Therefore, Muse cells or cell populations containing Muse cells, for example, can be isolated from body tissue using these antigen markers. Muse cells are also stress-tolerant, and can be concentrated from mesenchymal tissue or cultured mesenchymal cells by different types of stress treatments. A cell fraction with Muse cells enriched by stress treatment may be used as the cell preparation of the present invention. The methods of separation and identification of Muse cells, and their features, are disclosed in detail in International Patent Publication No. WO2011/007900. Also, as reported by Wakao et al. (2011, ibid.), when mesenchymal cells are cultured from the bone marrow or skin and used as a parent population of Muse cells, all of the SSEA-3 positive cells are also CD105-positive. Consequently, when Muse cells are isolated from mesenchymal tissue of a body or cultured mesenchymal stem cells for the cell preparation and pharmaceutical composition of the present invention, the Muse cells may be used after purification with SSEA-3 alone as the antigen marker. Throughout the present specification, pluripotent stem cells (Muse cells) or a cell population containing Muse cells, isolated from mesenchymal tissue of a body or cultured mesenchymal tissue using SSEA-3 as the antigen marker, and which can be used in a cell preparation and pharmaceutical composition for amelioration and/or treatment of brain disorder associated with fetal growth retardation, may be referred to simply as "SSEA-3 positive cells". Also throughout the present specification, "non-Muse cells" refers to cells that are present in mesenchymal tissue of a body or cultured mesenchymal tissue, and are the remainder of "SSEA-3 positive cells". In the Examples provided below, the non-Muse cells used are cell populations obtained by removing the SSEA-3 and CD105-positive cells from MSC by the method described in International Patent Publication No. WO2011/007900 for separation and identification of human Muse cells.

In brief, Muse cells or a cell population containing Muse cells can be isolated from body tissue (for example, mesenchymal tissue) using only antibody for the cell surface marker SSEA-3, or using antibodies for both SSEA-3 and CD105. The term "body" here means "mammalian body". According to the present invention, the "body" does not include a fertilized ovum or an embryo at a developmental stage before the blastocyst stage, but it does include an embryo at the developmental stage from the blastocyst stage onward, including the fetus or blastocyst. The mammal is not limited and may be a primate such as human or monkey, a rodent such as a mouse, rat, rabbit or guinea pig, or a cat, dog, sheep, pig, cow, horse, donkey, goat or ferret. The Muse cells to be used in the cell preparation and pharmaceutical composition of the present invention are clearly distinguished from embryonic stem cells (ES cells) or iPS cells based on separation from body tissue using a direct marker. The term "mesenchymal tissue" refers to tissue from the bone, synovial membrane, fat, blood, bone marrow, skeletal muscle, dermis, ligament, tendon, dental pulp, umbilical cord or umbilical cord blood, or tissues present in various organs. For example, the Muse cells may be obtained from the bone marrow or skin or adipose tissue. Preferably, mesenchymal tissue of a body is harvested and the Muse cells are isolated from the tissue and used. The separating means mentioned above may be used to separate Muse cells from cultured mesenchymal cells such as fibroblasts or bone marrow-derived MSCs. The Muse cells to be used for the cell preparation and pharmaceutical composition of the present invention may be either autologous or allogenic with respect to the recipient.

As mentioned above, Muse cells or a cell population containing Muse cells can be isolated from body tissue using SSEA-3 positivity, or double positivity for SSEA-3 and CD105, as indicators, but human adult skin is known to include various types of stem cells and progenitor cells. However, Muse cells are not identical to these cells. Such stem cells and progenitor cells include skin-derived precursors (SKP), neural crest stem cells (NCSC), melanoblasts (MB), perivascular cells (PC), endothelial precursor cells (EP) and adipose-derived stem cells (ADSC). Muse cells can be separated out as being "non-expressing" for the markers unique to these cells. More specifically, Muse cells can be separated by using non-expression for at least one, and for example, 2, 3, 4, 5, 6, 7, 8, 9, 10 or 11 among 11 markers selected from the group consisting of CD34 (EP and ADSC marker), CD117 (c-kit) (MB marker), CD146 (PC and ADSC marker), CD271 (NGFR) (NCSC marker), NG2 (PC marker), vWF factor (von Willebrand factor) (EP marker), Sox10 (NCSC marker), Snai1 (SKP marker), Slug (SKP marker), Tyrp1 (MB marker) and Dct (MB marker). As a non-limitative example, non-expression of CD117 and CD146 may be used as the indicator for separation, non-expression of CD117, CD146, NG2, CD34, vWF and CD271 may be used as the indicator, or non-expression of all of the aforementioned 11 markers may be used as the indicator for separation.

The Muse cells having the aforementioned features to be used for the cell preparation and pharmaceutical composition of the present invention may have at least one property selected from the group consisting of the following:

(i) low or non-existent telomerase activity;
(ii) having the ability to differentiate into any of the three germ layers;
(iii) exhibiting no neoplastic proliferation; and
(iv) having self-renewal ability.

According to one aspect of the present invention, the Muse cells to be used for the cell preparation and pharmaceutical composition of the present invention have all of these properties. As regards (i), "low or non-existent telomerase activity", this refers to low or non-detectable telomerase activity when using a TRAPEZE XL telomerase detection kit (Millipore), for example. "Low" telomerase activity is, for example, either telomerase activity on the same level as human fibroblasts, which are somatic cells, or telomerase activity of 1/5 and preferably no greater than 1/10 of that of Hela cells. In regard to (ii), the Muse cells have the ability to differentiate into the three germ layers (endoderm, mesoderm and ectoderm) *in vitro* and *in vivo,* and by induction culturing *in vitro,* for example, they can differentiate into hepatocytes, neurons, skeletal muscle cells, smooth muscle cells, osteocytes or adipocytes. They may also exhibit the ability to differentiate into the three germ layers in the case of transplanting *in vivo* into the testes. They also have the ability to migrate and engraft onto damaged organs (heart, skin, spine, liver, muscle, etc.), by administration into the body via intravenous injection, and differentiate into specific cells of the corresponding tissue. In regard to (iii), the Muse cells have the property of proliferating at a rate of about every 1.3 days in suspension culture, and growing in suspension culture from a single cell to form an embryoid-like cell mass, slow down the growth at about 14 days; however, when the embryoid-like cell mass is carried into adhesion culture, cell growth resumes and the proliferated cells spread out from the cell mass. They also have the property of not generating teratomas at least for 6 months after transplantation into the testes. In regard to (iv), Muse cells have self-renewal (auto-replicating) ability. The term "self-renewal" means that cells in the embryoid-like cell mass obtained by culturing a single Muse cell in suspension culture can be confirmed to differentiate into cells of all 3 germ layers, and also that when a single cell from the embryoid-like cell mass is again carried into a suspension culture, it forms a next generation embryoid-like cell mass, and reproduce differentiation into three germ layers as well as embryoid-like cell mass in the suspension culture can be confirmed. Self-renewal may be observed once or as several repeated cycles.

In addition, a cell fraction containing Muse cells to be used in the cell preparation of the present invention may be a cell fraction having the SSEA-3 positive and CD105-positive pluripotent stem cells concentrated, obtained by a method of applying external stress treatment to mesenchymal tissue of a body or cultured mesenchymal cells, causing the cells other than the external stress-resistant cells to die, and recovering the surviving cells, the cell fraction having at least one and preferably all of the following properties.

(i) SSEA-3 positivity;
(ii) CD105-positivity;
(iii) low or non-existent telomerase activity;
(iv) having the ability to differentiate into any of the three germ layers;
(v) exhibiting no neoplastic proliferation; and
(vi) having self-renewal ability.

The external stress may be any one or a combination of: protease treatment, culturing in a low oxygen concentration, culturing under low-phosphate conditions, culturing with low serum concentration, culturing under low nutritive conditions, culturing under exposure to heat shock, culturing at low temperature, freezing treatment, culturing in the presence of a hazardous substance, culturing in the presence of active oxygen, culturing under mechanical stimulation, culturing with agitating treatment, culturing with pressure treatment, or physical impact. For example, the treatment time with a protease is preferably a total of 0.5 to 36 hours to apply external stress to the cells. The protease concentration may be the concentration used when the cells adhering to a culture vessel are detached, when the cell mass is dispersed into individual cells, or when individual cells are recovered from tissue. The protease is preferably a serine protease, aspartic acid protease, cysteine protease, metalloprotease, glutamic acid protease or N-terminal threonine protease. The protease is also preferably trypsin, collagenase or dispase.

Muse cells having the aforementioned features, which are to be used in the cell preparation of the present invention, are administered to the body by intravenous administration or the like, after which they engraft onto damaged brain tissue, as described below. It is thought that the Muse cells differentiate into tissue-compatible cells thus ameliorating and/or treating brain disorder associated with fetal growth retardation.

(2) Preparation and use of cell preparation and pharmaceutical composition

Although not limited thereto, the cell preparation and pharmaceutical composition of the present invention may be obtained by suspending the Muse cells or a cell population containing Muse cells obtained by (1) above, in physiological saline or an appropriate buffer (for example, phosphate-buffered physiological saline). In this case, when the number of Muse cells isolated from autologous or allogenic tissue is low, the cells may be cultured before administration for growth until the prescribed cell density is obtained. As already reported (International Patent Publication No. WO2011/007900), Muse cells do not undergo neoplastic transformation, and therefore even if the cells recovered from body tissue are in undifferentiated form, they have low tumorigenicity and are safe. There are no particular restrictions on culturing of the recovered Muse cells, and it may be carried out in ordinary growth medium (for example, α-Minimal Essential Medium (α-MEM) containing 10% newborn calf serum). More specifically, referring to International Patent Publication No. WO2011/007900, suitable medium and additives (for example, antibiotics and serum) may be selected for culturing and growth of the Muse cells, and a solution containing the prescribed density of Muse cells may be prepared. When a cell preparation or pharmaceutical composition of the present invention is to be administered to a human patient, roughly several milliliters of bone marrow fluid may be harvested from human iliac bone, and for example, the bone marrow-derived MSCs may be cultured as adherent cells from the bone marrow fluid to increase them to a number of cells allowing separation of an effective therapeutic amount of Muse cells, after which the Muse cells may be separated out with SSEA-3 antigen marker as the indicator, and autologous or allogenic Muse cells prepared as a cell preparation. As an alternative example, Muse cells that have been separated using SSEA-3 antigen marker as the indicator, and the cells cultured to increase them to an effective therapeutic amount, may then be prepared as a cell preparation of autologous or allogenic Muse cells.

For use of the Muse cells in a cell preparation or pharmaceutical composition, dimethyl sulfoxide (DMSO) or serum albumin may be added to the cell preparation or pharmaceutical composition to protect the cells, and an antibiotic or the like may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like), or cells or components other than Muse cells that are present among MSCs, may be added to the cell preparation or pharmaceutical composition. A person skilled in the art may add such factors and chemical agents to the cell preparation and pharmaceutical composition in appropriate concentrations.

The number of Muse cells in the cell preparation and pharmaceutical composition to be prepared may be appropriately adjusted so as to obtain the desired effect for amelioration and/or treatment of brain disorders associated with fetal growth retardation (for example, improvement in motor quality or improvement in neurological development), in consideration of the target gender, age and body weight, the state of the affected area, and the state of the cells to be used. In Example 1 below, pregnant rats were fitted with an ameroid constrictor (AC) in the uterus, producing fetal chronic ischemia to create an intrauterine development model. The rat models born from the parents, having undergone fetal growth retardation, were treated and the therapeutic effect of Muse cell grafts was examined. When rat models with body weight of approximately 15 to 20 g were administered SSEA3-positive cells at $1 \times 10^4$ cells/rat, a very excellent effect was obtained. Based on these results, it is expected that for human neonates (within 28 days after birth), infants (less than 1 year after birth) or children (1 to 6 years after birth), administration of cells in an amount of approximately $1 \times 10^5$ cells/kg to approximately $1 \times 10^8$ cells/kg per individual, based on weight, should yield an excellent effect. For example, for a neonate with a body weight of about 1,000 g, an estimated dose of about $1 \times 10^5$ cells/individual to about $1 \times 10^8$ cells/individual would be expected to be effective. However, in order to avoid obstruction by administration of cells into blood vessels, the SSEA-3 positive cells may be added to the cell preparation at no greater than $1 \times 10^7$ cells/individual, for example, as the amount per single administration. Here, "individual" includes, but is not limited to, a rat or human. The cell preparation and pharmaceutical composition of the invention may be administered several times (for example, 2 to 10 times) at appropriate intervals (for example, twice a day, once a day, twice a week, once a week or once every 2 weeks), until the desired therapeutic effect is obtained. Therefore, the therapeutically effective amount, while depending on the condition of the subject, is preferably a dose of $1 \times 10^4$ cells to $1 \times 10^7$ cells per individual, administered 1 to 10 times, for example. The total amount of administration per rat is not limited, and may be $1 \times 10^5$ cells to $1 \times 10^8$ cells, $1 \times 10^5$ cells to $5 \times 10^7$ cells, $1 \times 10^5$ cells to $1 \times 10^7$ cells, $1 \times 10^5$ cells to $5 \times 10^6$ cells, $1 \times 10^5$ cells to$1 \times 10^6$ cells, $5 \times 10^5$ cells to $1 \times 10^8$ cells, $5 \times 10^5$ cells to $5 \times 10^7$ cells, $5 \times 10^5$ cells to $1 \times 10^7$ cells, $5 \times 10^5$ cells to $5 \times 10^6$ cells, $5 \times 10^5$ cells to $1 \times 10^6$ cells, $1 \times 10^6$ cells to $1 \times 10^8$ cells, $1 \times 10^6$ cells to $5 \times 10^7$ cells, $1 \times 10^6$ cells to $1 \times 10^7$ cells or $1 \times 10^6$ cells to $5 \times 10^6$ cells.

The cell preparation and pharmaceutical composition of the invention is to be used for amelioration and treatment of a brain disorder associated with fetal growth retardation, where the period of administration is assumed to be after confirming neurological symptoms and growth retardation, although it may be immediately after birth in cases of severe fetal growth retardation. That is, it is preferably administered immediately after injury, although the effect of the cell preparation of the invention may be expected at a later period after injury, such as 1 hour, 1 days, 1 week, 1 month, 3 months or 6 months after injury, for example. Furthermore, since the Muse cells to be used have been confirmed in experiments by the present inventors to not elicit an immune response even when heterologously derived, they may be suitably administered until the desired effect for amelioration and treatment of brain disorder associated with fetal growth retardation is obtained. In Examples 2 to 4 below, behavioral evaluation was conducted to determine improvement in brain disorder associated with fetal growth retardation by Muse cells using rat models born from the intrauterine development models described above (hereunder also referred to as "fetal growth retardation rat models"), and a marked improvement in brain disorder was found as a result.

The Muse cells to be used in the cell preparation and pharmaceutical composition of the present invention have the property of accumulating at disease sites. For administration of the cell preparation or pharmaceutical composition, therefore, there is no restriction on the mode of administration (for example, intraperitoneal, intramuscular, or local injection at the affected area), or the type of blood vessel for administration (vein or artery). The method for confirming whether the administered Muse cells have reached and engrafted to the affected site may be, for example, creation of Muse cells previously subjected to gene transfer so as to express a fluorescent protein (for example, green fluorescent protein (GFP)), and after administration to the body, observation may be carried out using a system that can detect fluorescence (for example, an $IVIS^R$ Imaging System (product of Pharma International, Inc.)), to confirm of the dynamics of the Muse cells. Since the Muse cells to be used in the cell preparation and pharmaceutical composition of the present invention are human-derived, they are heterogenous with respect to rats. In an experiment in which allogenic cells are administered to an animal model, an immunosuppressive agent (such as cyclosporin) may be administered either before or simultaneously with administration of the allogenic cells to suppress *in vivo* rejection of the heterogenous cells.

3. Creation of intrauterine development model with fetal growth retardation

For the purpose of the present specification, in order to examine the amelioration and treatment effect on brain disorders associated with fetal growth retardation (abnormal quality of movement or abnormal neurological development, for example) by the cell preparation and pharmaceutical composition of the invention, it is possible to create an intrauterine development model with growth retardation in pregnant rats and to use the rats with brain disorders that are born from these rats. The intrauterine development model is not restricted, and for example, an ameroid constrictor (AC) (inner diameter: 0.45 mm, 0.40 mm, etc.) or a micro coil (inner diameter: 0.24 mm) may be fitted at 4 locations of the uterine artery of the rats on the 17th day of pregnancy, to cause chronic ischemia of the fetus. The AC used includes casein in the interior and has an internal slit and a hole at the center, while being covered with a wide-area titanium ring. The action mechanism for producing chronic ischemia is that the casein inside the AC gradually expands by water absorption, and as the hole decreases in size the diameter of the uterine artery section on which the AC has been fitted is narrowed, thus creating ischemia. Rats that are usable in the intrauterine development model are not restricted, and generally include Wistar/ST rats and Sprague Dawley (SD) rats.

4. Amelioration and treatment effect by Muse cells in fetal growth retardation rat model.

The cell preparation and pharmaceutical composition of the invention can ameliorate and/or treat brain disorders associated with fetal growth retardation in mammals including humans. When a fetal growth retardation rat model born from an intrauterine development model created as described above according to the invention is used, improvement in symptoms by Muse cells in experimental rats with brain disorder associated with fetal growth retardation can be examined and the effect of the Muse cells can be evaluated. The specific method of evaluation may be carried out using a common experiment system used for evaluation of brain function using rats, and it may be behavioral evaluation by a negative geotaxis test, Rota Rod Test, Open Field Test, Shuttle Avoidance Test, Novel Object Recognition Test, (Cat Walk Test or Morris Water Maze Test, for example.

A "negative geotaxis test" is a method of evaluation by measurement of the time until initial behavior of a test animal after separation from stimulation. For example, the rat may be placed on a tilted plate with head facing down and measurement made of the movement reaction time until the head is raised back up, thus allowing the reflex time to be used as an index of improved brain function.

The "Rota Rod Test" is a test using an apparatus that measures the coordination of motor function and static sense of a test animal. Specifically, the test animal is placed on an apparatus having a rotating rod that is capable of constant acceleration, and it is gradually accelerated from slow rotation. The time during which the test animal can walk to match the rotation without falling off from the rotating rod is determined. Repeated testing allows the motor learning function to be examined.

The "Open Field Test" is based on placement of the test animal in a novel fixed space (for example, a 60 (W) × 60 (D) × 40 (H) cm box) and observation of the emotional behavior of the test animal, such as exploratory behavior. The observer may actually record the behavior of the test animal while photographing it with a video camera, extracting data and evaluating the indicators of activity/affectivity (for example, traveling distance, rest time and center dwelling rate).

The "Shuttle Avoidance Test" is a test using an apparatus for observation of conditioned response behavior of a test animal to sound or light, and it allows evaluation of learning disability. With the test animal in one room, a sound is produced or a light is flashed, and then a current is circulated through the floor and the test animal escapes to another room to avoid the electrical stimulation. Repeating this procedure allows evaluation of the presence or absence of learning disability in the test animal.

In the "Novel Object Recognition Test", the test animal is allowed to freely explore a space in which two objects have been placed, and then one of the objects is replaced with a new object and the change in exploration time by the test animal for the new object is measured, to allow evaluation of memory learning and visual cognitive memory.

In the "Cat Walk Test", the test animal is caused to walk on a transparent glass plate with interior LED lighting, and video images of the foot soles taken from below with only the contact surface illuminated, based on the principle of total internal reflection, are analyzed by personal computer software, to allow evaluation of leg movement and the walking condition of the test animal.

In the "Morris Water Maze Test", the rat is caused to swim in a water tank having a platform at water surface level at one location, and the time or distance until it reaches the platform are evaluated to allow evaluation of spatial cognition.

The present invention will now be explained in more specific detail through the following examples, with the understanding that the invention is in no way limited by the examples.

## EXAMPLES

### Example 1: Creation of fetal growth retardation rat model and administration of Muse cells

The protocol for the experimental animals used in this research were approved by the Committee for Animal Experiments of Nagoya University, Faculty of Medicine. SD pregnant rats were obtained from Japan SLC, Inc. (Shizuoka Prefecture, Japan), and were placed in cages with free access to food and water and reared with a 12-hour light/dark cycle. The animal room and experimental space were kept at a constant 23°C.

Pregnant 17-day-old mother rats were fitted with ameroid constrictors (AC) (inner diameter: 0.45 mm or 0.40 mm) at 4 locations of the uterine artery, producing chronic ischemia in the fetuses to create an intrauterine development model. The baby rats born from the mothers were used as a fetal growth retardation model. The born rats were determined to have fetal growth retardation by periodically measuring the body weight of the rats after birth, and comparing them to the body weights of normal control rats, to confirm reduced increase in body weight change (data not shown).

On the 4th day after birth, Muse cells ($1 \times 10^4$ cells/rat) were administered through the left cervical vein, to prepare a "Muse cell-administered group". As control groups, rats administered non-Muse cells were prepared as a "non-Muse cell-administered group", and rats administered STEM-CELLBANKER$^R$ cell preservation solution were prepared as a "vehicle group". Rats born from mother rats that had been subjected to sham surgery were used as a "sham group".

Example 2: Evaluation of improvement in brain function (1 month after birth) using AC (0.45 mm)

(1) Negative geotaxis test

Improvement in behavioral disorder due to brain disorder associated with fetal growth retardation, by grafting of Muse cells, was examined by a negative geotaxis test. Fetal growth retardation rat models (hereunder referred to simply as "rats") were placed on a 30-degree tilted plate with heads oriented downward, and the time (seconds) until the rats raised their heads in reflex was measured, evaluating the effect of each treatment based on the different in time. The number of rats in each group was n = 31 in the sham group, n = 18 in the vehicle group, n = 19 in the Muse cell-administered group and n = 18 in the non-Muse cell group. The rats used for the test were 8 to 11 days old, and the test was conducted once per day for 4 successive days. The negative geotaxis test for each group is shown in Fig. 1A. The ordinate represents time (seconds) until the rats raised their heads in reflex, and it is seen that in all cases, time to raising of the head shortened with elapsing days after birth. In the test on the 8th day after birth, the Muse cell-administered group had a shorter time to establishing reflex compared to the control groups (vehicle group and non-Muse cell-administered group), while an even more marked effect was observed on the 9th day after birth, as it reached the same time to establishing reflex as the sham group. This demonstrated that Muse cells exhibit a significant effect for behavior improvement in rats with brain disorders.

In addition, the time (seconds) measured for all 4 days was totaled for each rat, and the data divided by 4 were replotted as average measured times, as shown in Fig. 1B. According to these results, the Muse cell-administered group had a shorter time to establishing reflex compared to the control groups (vehicle group and non-Muse cell-administered group), while tending to reach the same time to establishing reflex as the sham group. This demonstrated that Muse cells exhibit a significant effect for behavior improvement in rats with brain disorders.

In the negative geotaxis test described above, the vehicle group was a group with only a cell preservation solution (STEM-CELLBANKER$^{R}$) added, but for the 9-day-old rats, the same test was conducted using the cell preservation solution instead of Hank's Balanced Salt Solution (HBSS), which was the base solution for the Muse cell group and non-Muse cell group (Fig. 7). Fig. 7 shows the results of scoring from 0 to 5 points on the following scale.

0 = Unresponsive
1 = Not reached within 60 seconds, or falling partway through
2 = Reached before 45 seconds
3 = Reached before 30 seconds
4 = Reached before 15 seconds
5 = Reached before 0 seconds

As is clear from the results in Fig. 7, significant improvement was observed in the Muse cell-administered group compared to the vehicle group, similar to the sham group.

(2) Rota Rod test

A commonly known apparatus (Rat Rota-Rod 47700 by UGO Basile) was used for measurement of the motor function coordination and static sense of test animals, to examine the improvement in brain disorder by transplantation of the Muse cells. The test evaluation measures the time until the rats fall from a rotating stage, with repetition of the test (total of 4 times), and with the indicator of improvement in motor learning function being lengthening of the time until falling. The results are shown in Fig. 2. Upon continuous measurement for 2 days, the Muse cell-administered group was found to have lengthened falling time on all days compared to the non-Muse cell-administered group and vehicle group. These results suggest that Muse cells can improve motor learning disability function, as a type of brain disorder.

For the Rota Rod test, a sham group was added to the control groups, and the vehicle group was a group with only a cell preservation solution (STEM-CELLBANKER$^{R}$) added, conducting the same test using this instead of Hank's Balanced Salt Solution (HBSS). The data calculated for each run of the test are shown at left in Fig. 8, and the average values after totaling the measured values for each run are shown at right in Fig. 8. The results shown at right in Fig. 8 suggest that the time until falling of the rats (1 month after birth) in the Muse cell group and non-Muse cell group was significantly extended compared to the vehicle group.

This test used rats at one month after birth, but the same test was also conducted using rats at 5 months after birth (Fig. 10). The data calculated for each run of the test are shown at left in Fig. 10, and the average values after totaling the measured values for each run are shown at right in Fig. 10. The time to falling in the Muse cell-administered group was significantly extended beyond that in the vehicle group, to about the same degree as the sham group. The falling time was also significantly shortened in the non-Muse cell-administered group compared to the Muse cell-administered group and sham group. Even with rats examined for a long period beyond one month after birth, brain disorder was still shown to be significantly improved by administration of Muse cells.

Example 3: Evaluation of improvement in brain function (4 months after birth) using AC (0.45 mm)

(1) Open field test

Rats were placed in a box having a novel fixed space, and the emotional behavior (hyperactivity, etc.) of the test animals was observed. The behavior of the test animals was photographed with a video camera (ANY-maze Video Tracking Software, Muromachi Kikai Co., Ltd.), and the traveling distance within a prescribed time period (Fig. 3A), the time immobile (Fig. 3B) and the mobile episodes (Fig. 3C) of the test animals were measured. Rats placed in a novel space have longer traveling distance due to anxiety, but the traveling distance is reduced when they adapt to the space. Although ameliorative effects were seen in both the Muse cell-administered group and non-Muse cell-administered group compared to the rat group, the effect was more notable with the Muse cell-administered group. This suggested that administration of Muse cells can improve emotional behavior of test animals.

This test used rats at 4 months after birth, but the same test was also conducted using rats in a longer period (5 months after birth) (Fig. 11). The vehicle group was observed to be more overactive than the other groups, frequently moving to bright areas. This tendency was also observed on the 2nd day and 3rd day. No such overactivity was observed in the other groups (sham group, Muse cell-administered group and non-Muse cell-administered group), however.

(2) Y maze test

A Y maze apparatus was used to evaluate the spontaneous movement activity and spatial working memory of the rats (1 month after birth). The number of times that they were introduced into different arms three consecutive times was divided by the value of the number of the total number of times introduced into arms minus 1, and the ratio (alternating response) was digitized and plotted for each group (Fig. 9). The rats in the Muse cell-administered group, similar to the sham group, had significantly improved spatial memory and cognition compared to the vehicle group, but no significant improvement was observed in the non-Muse cell-administered group.

Example 4: Evaluation of improvement in brain function (1 month after birth) using AC (0.40 mm)

(1) Negative geotaxis test

A negative geotaxis test was conducted in the same manner as Example 2. The results are shown in Figs. 4 and 5. Fig. 4 shows the results of evaluation based on the time (seconds) until reflexive raising of the head, as in Fig. 1, and Fig. 5 shows the results of scoring from 0 to 5 points based on the following scale.

0 = Unresponsive
1 = Not reached within 60 seconds, or falling partway through
2 = Reached before 60 seconds
3 = Reached before 45 seconds
4 = Reached before 30 seconds
5 = Reached before 15 seconds

Upon evaluation based on the measured time, the tendency seen with an AC inner diameter of 0.45 mm (Fig. 1A) was also seen with an AC inner diameter of 0.40 mm (Fig. 4A). In comparison to the control groups, the Muse cell-administered group had notable improvement in reflex movement disorder compared to the vehicle group, with a significant effect also being observed in comparison to the non-Muse cell-administered group. In addition, a significant effect was exhibited in the Muse cell-administered group even when replotted as average time for 4 days for each group as in Fig. 1B (Fig. 4B).

Fig. 5 shows the results of evaluation of this negative geotaxis test using the scoring shown above. As in Fig. 4, marked improvement in reflex movement disorder was seen in the Muse cell-administered group, with a similar tendency observed in the other groups as well. This suggested that administration of Muse cells can improve reflex movement of test animals.

(2) Novel object recognition test

A novel object recognition test was conducted. The results are shown in Fig. 6. A commonly employed apparatus was used for the novel object recognition test (ANY-maze Video Tracking Software, product of Muromachi Kikai Co., Ltd.). Rats were allowed to freely explore a space in which two objects (familiar objects) had been placed, for a fixed period of time, after which one of the objects was replaced with a novel object and the difference in exploration time of the rat for the new object was measured. The recognition index (discrimination index) was used as the index for the evaluation. Specifically, calculation was by $RI = (TN \times 100)/(TN + TF)$ (where TF: exploration time for familiar object, TN: exploration time for novel object). A higher RI value indicates higher interest for the object. The results are shown in Fig. 6. These test results suggested the possibility that memory learning and visual cognitive memory can be improved by administration of Muse cells.

(3) Rota Rod Test

A Rota Rod test was conducted in the same manner as Example 2. The results are shown in Fig. 12.

The test was conducted using rats at 5 months after birth, in the same manner as described above. The results are shown in Fig. 13. The data measured for each run of the test are shown at left in Fig. 12 and at left in Fig. 13, and the average values after totaling the measured values for each run are shown at right in Fig. 12 and at right in Fig. 13. With the rats at 1 month after birth, the Muse cell-administered group had a significantly extended time until falling compared to the vehicle group, at about the same degree as the sham group, while this effect was not seen with the non-Muse cell-administered group (right in Fig. 12). A notable effect was also observed in the Muse cell-administered group even with rats in a longer period (5 months), with a significant effect being seen in comparison to the non-Muse cell-administered group (right in Fig. 13).

INDUSTRIAL APPLICABILITY

The cell preparation and pharmaceutical composition of the invention can be administered to fetal growth retardation rat models for amelioration and treatment of brain disorders associated with fetal growth retardation, including abnormal quality of movement and abnormal neurological development.

## Claims

**1.** A cell preparation for use in amelioration and/or treatment of a brain disorder associated with fetal growth retardation due to hypoxemia caused by placental or umbilical cord factors, or due to complex and chronic factors such as circulatory failure and malnutrition, comprising an effective amount of pluripotent stem cells positive for SSEA-3 isolated from mesenchymal tissue or cultured mesenchymal cells of a living organism,
wherein the brain disorder associated with fetal growth retardation is selected from the group consisting of abnormal quality of movement, abnormal neurological development, cerebral palsy, cognitive impairment, and behavior disorder.

**2.** The cell preparation for use according to claim 1, comprising a cell fraction wherein pluripotent stem cells positive for SSEA-3 have been concentrated by external stress treatment.

**3.** The cell preparation for use according to claim 1 or 2, wherein the pluripotent stem cells are CD105-positive.

**4.** The cell preparation for use according to any one of claims 1 to 3, wherein the pluripotent stem cells are CD117-negative and CD146-negative.

**5.** The cell preparation for use according to any one of claims 1 to 4, wherein the pluripotent stem cells are CD117-negative, CD146-negative, NG2-negative, CD34-negative, vWF-negative and CD271-negative.

**6.** The cell preparation for use according to any one of claims 1 to 5, wherein the pluripotent stem cells are CD34-negative, CD117-negative, CD146-negative, CD271-negative, NG2-negative, vWF-negative, Sox10-negative, Snai1-negative, Slug-negative, Tyrp1-negative and Dct-negative.

**7.** The cell preparation for use according to any one of claims 1 to 6, wherein the pluripotent stem cells have all of the following properties:

(i) low or non-existent telomerase activity;
(ii) having the ability to differentiate into any of the three germ layers;
(iii) exhibiting no neoplastic proliferation; and
(iv) having self-renewal ability.

**8.** The cell preparation for use according to any one of claims 1 to 7, wherein the pluripotent stem cells have the ability to engraft to brain tissue.

**9.** The cell preparation for use according to any one of claims 1 to 8 , which is to be administered to a human neonate, infant or child with the pluripotent stem cells at from approximately $1 \times 10^5$ cells/individual to approximately $1 \times 10^8$ cells/individual, as the therapeutically effective amount.

**10.** The cell preparation for use according to any one of claims 1 to 9, which is to be administered to a human neonate, infant or child with the pluripotent stem cells in an amount of cells per body weight of approximately $1 \times 10^5$ cells/kg to approximately $1 \times 10^8$ cells/kg per target individual, as the therapeutically effective amount.

## Patentansprüche

**1.** Zellpräparat zur Verwendung bei der Besserung und/oder Behandlung einer Hirnstörung im Zusammenhang mit einer fetalen Wachstumsverzögerung aufgrund von Hypoxämie verursacht durch Plazenta- oder Nabelschnurfaktoren oder aufgrund komplexer und chronischer Faktoren, wie z. B. Kreislaufversagen und Unterernährung, welches eine wirksame Menge an pluripotenten Stammzellen umfasst, die positiv für SSEA-3 sind und aus mesenchymalem Gewebe oder kultivierten mesenchymalen Zellen eines lebenden Organismus isoliert wurden,
wobei die mit der fetalen Wachstumsverzögerung im Zusammenhang stehende Hirnstörung ausgewählt ist aus der Gruppe bestehend aus abnormaler Bewegungsqualität, abnormaler neurologischer Entwicklung, Zerebralparese, kognitiver Beeinträchtigung und Verhaltensstörung.

**2.** Zellpräparat zur Verwendung nach Anspruch 1, welches eine Zellfraktion umfasst, wobei pluripotente Stammzellen, die positiv für SSEA-3 sind, durch eine externe Stressbehandlung konzentriert wurden.

**3.** Zellpräparat zur Verwendung nach Anspruch 1 oder 2, wobei die pluripotenten Stammzellen CD105-positiv sind.

**4.** Zellpräparat zur Verwendung nach einem der Ansprüche 1 bis 3, wobei die pluripotenten Stammzellen CD117-negativ und CD146-negativ sind.

**5.** Zellpräparat zur Verwendung nach einem der Ansprüche 1 bis 4, wobei die pluripotenten Stammzellen CD117-negativ, CD146-negativ, NG2-negativ, CD34-negativ, vWF-negativ und CD271-negativ sind.

**6.** Zellpräparat zur Verwendung nach einem der Ansprüche 1 bis 5, wobei die pluripotenten Stammzellen CD34-negativ, CD117-negativ, CD146-negativ, CD271-negativ, NG2-negativ, vWF-negativ, Sox10-negativ, Snai1-negativ, Slug-negativ, Tyrp1-negativ und Dct-negativ sind.

**7.** Zellpräparat zur Verwendung nach einem der Ansprüche 1 bis 6, wobei die pluripotenten Stammzellen alle der folgenden Eigenschaften aufweisen:

(i) geringe oder nicht vorhandene Telomerase-Aktivität;
(ii) die Fähigkeit, sich in jede der drei Keimschichten zu differenzieren;
(iii) keine neoplastische Proliferation; und
(iv) die Fähigkeit zur Selbsterneuerung.

**8.** Zellpräparat zur Verwendung nach einem der Ansprüche 1 bis 7, wobei die pluripotenten Stammzellen die Fähigkeit aufweisen, in Hirngewebe einzuwachsen.

**9.** Zellpräparat zur Verwendung nach einem der Ansprüche 1 bis 8, welches einem menschlichen Neugeborenen, Säugling oder Kind mit den pluripotenten Stammzellen in einer Menge von etwa $1 \times 10^5$ Zellen/Individuum bis etwa $1 \times 10^8$ Zellen/Individuum als die therapeutisch wirksame Menge verabreicht werden soll.

**10.** Zellpräparat zur Verwendung nach einem der Ansprüche 1 bis 9, welches einem menschlichen Neugeborenen, Säugling oder Kind mit den pluripotenten Stammzellen in einer Menge von Zellen pro Körpergewicht von etwa $1 \times 10^5$ Zellen/kg bis etwa $1 \times 10^8$ Zellen/kg pro Zielindividuum als die therapeutisch wirksame Menge verabreicht werden soll.

## Revendications

**1.** Préparation cellulaire destinée à être utilisée dans l'amélioration et/ou le traitement d'un trouble cérébral associé à un retard de croissance fœtale dû à une hypoxémie provoquée par des facteurs placentaires ou du cordon ombilical, ou dû à des facteurs complexes et chroniques tels qu'une défaillance circulatoire et une malnutrition, comprenant une quantité efficace de cellules souches pluripotentes positives pour SSEA 3 isolées de tissu mésenchymateux ou de cellules mésenchymateuses cultivées d'un organisme vivant,
dans laquelle le trouble cérébral associé à un retard de croissance fœtale est choisi dans le groupe constitué par une qualité motrice anormale, un développement neurologique anormal, une paralysie cérébrale, un trouble cognitif et un trouble du comportement.

**2.** Préparation cellulaire destinée à être utilisée selon la revendication 1, comprenant une fraction cellulaire dans laquelle des cellules souches pluripotentes positives pour SSEA 3 ont été concentrées par traitement du stress externe.

**3.** Préparation cellulaire destinée à être utilisée selon la revendication 1 ou 2, dans laquelle les cellules souches pluripotentes sont CD105 positives.

**4.** Préparation cellulaire destinée à être utilisée selon l'une quelconque des revendications 1 à 3, dans laquelle les cellules souches pluripotentes sont CD117 négatives et CD146 négatives.

**5.** Préparation cellulaire destinée à être utilisée selon l'une quelconque des revendications 1 à 4, dans laquelle les cellules souches pluripotentes sont CD117 négatives, CD146 négatives, NG2 négatives, CD34 négatives, vWF négatives et CD271 négatives.

**6.** Préparation cellulaire destinée à être utilisée selon l'une quelconque des revendications 1 à 5, dans laquelle les cellules souches pluripotentes sont CD34 négatives, CD117 négatives, CD146 négatives, CD271 négatives, NG2 négatives, vWF négatives, Sox10 négatives, Snai1 négatives, Slug négatives, Tyrp1 négatives et Dct négatives.

**7.** Préparation cellulaire destinée à être utilisée selon l'une quelconque des revendications 1 à 6, dans laquelle les cellules souches pluripotentes possèdent toutes les propriétés suivantes :

(i) activité de la télomérase faible ou inexistante ;
(ii) possédant la capacité de se différencier en l'un quelconque des trois feuillets embryonnaires ;
(iii) ne présentant aucune prolifération néoplasique ; et
(iv) possédant une capacité d'auto renouvellement.

**8.** Préparation cellulaire destinée à être utilisée selon l'une quelconque des revendications 1 à 7, dans laquelle les cellules souches pluripotentes possèdent la capacité de se greffer au tissu cérébral.

**9.** Préparation cellulaire destinée à être utilisée selon l'une quelconque des revendications 1 à 8, qui est destinée à être administrée à un nouveau-né, un nourrisson ou un enfant humain avec les cellules souches pluripotentes à hauteur d'environ $1 \times 10^5$ cellules/individu à environ $1 \times 10^8$ cellules/individu, en tant que quantité thérapeutiquement efficace.

**10.** Préparation cellulaire destinée à être utilisée selon l'une quelconque des revendications 1 à 9, qui est destinée à être administrée à un nouveau-né, un nourrisson ou un enfant humain avec les cellules souches pluripotentes en une quantité de cellules par poids corporel d'environ $1 \times 10^5$ cellules/kg à environ $1 \times 10^8$ cellules/kg par individu cible, en tant que quantité thérapeutiquement efficace.

FIG. 1A

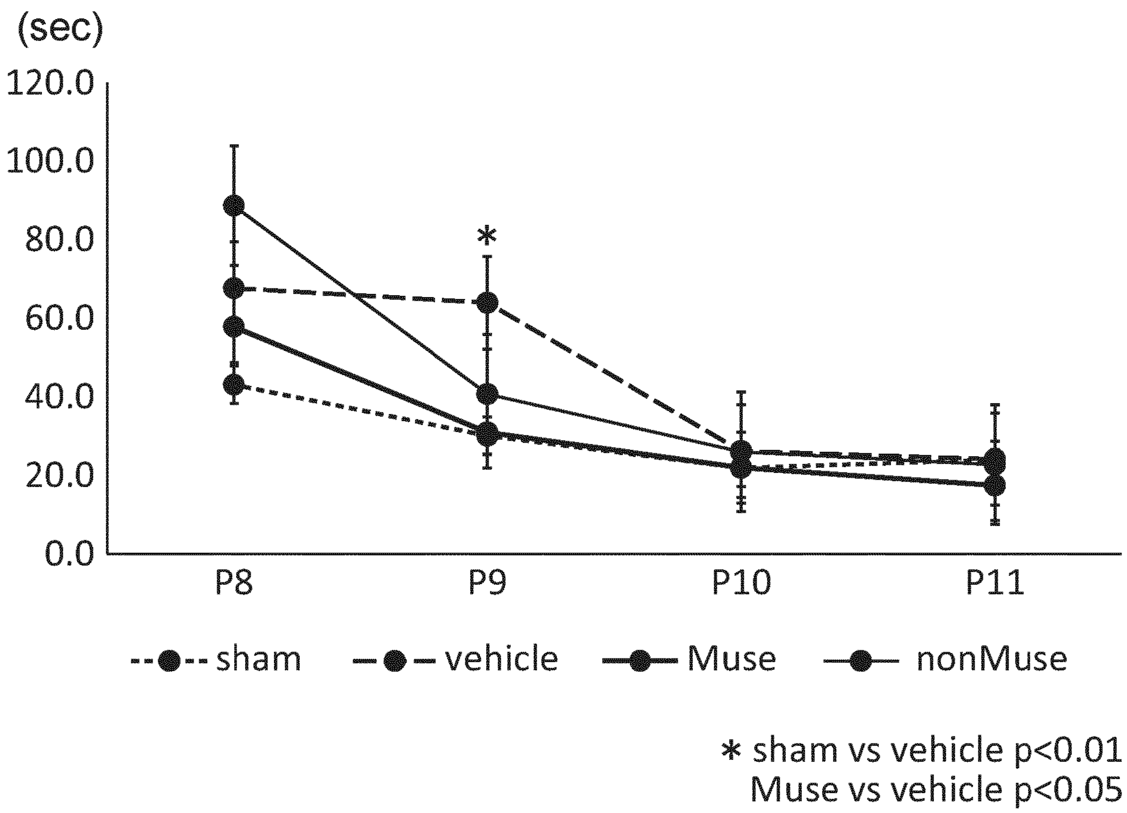
(sec)
120.0
100.0
80.0
60.0
40.0
20.0
0.0
*
P8
P9
P10
P11
sham
vehicle
Muse
nonMuse
* sham vs vehicle p<0.01
Muse vs vehicle p<0.05

FIG. 1B

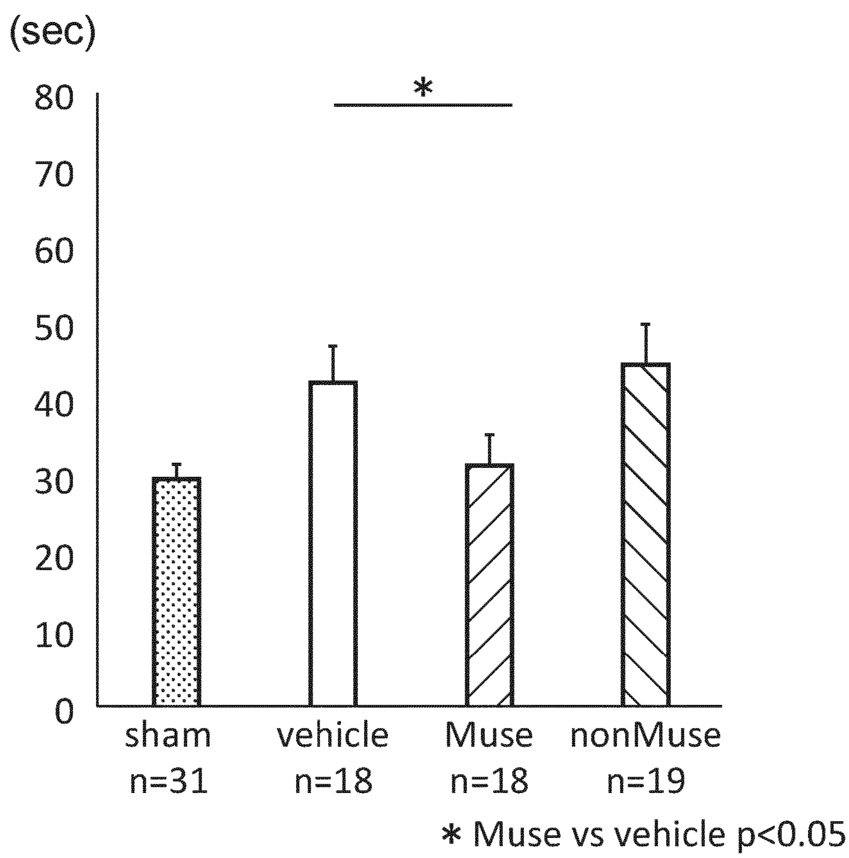
(sec)
80
70
60
50
40
30
20
10
0
*
sham
n=31
vehicle
n=18
Muse
n=18
nonMuse
n=19
* Muse vs vehicle p<0.05

FIG. 2

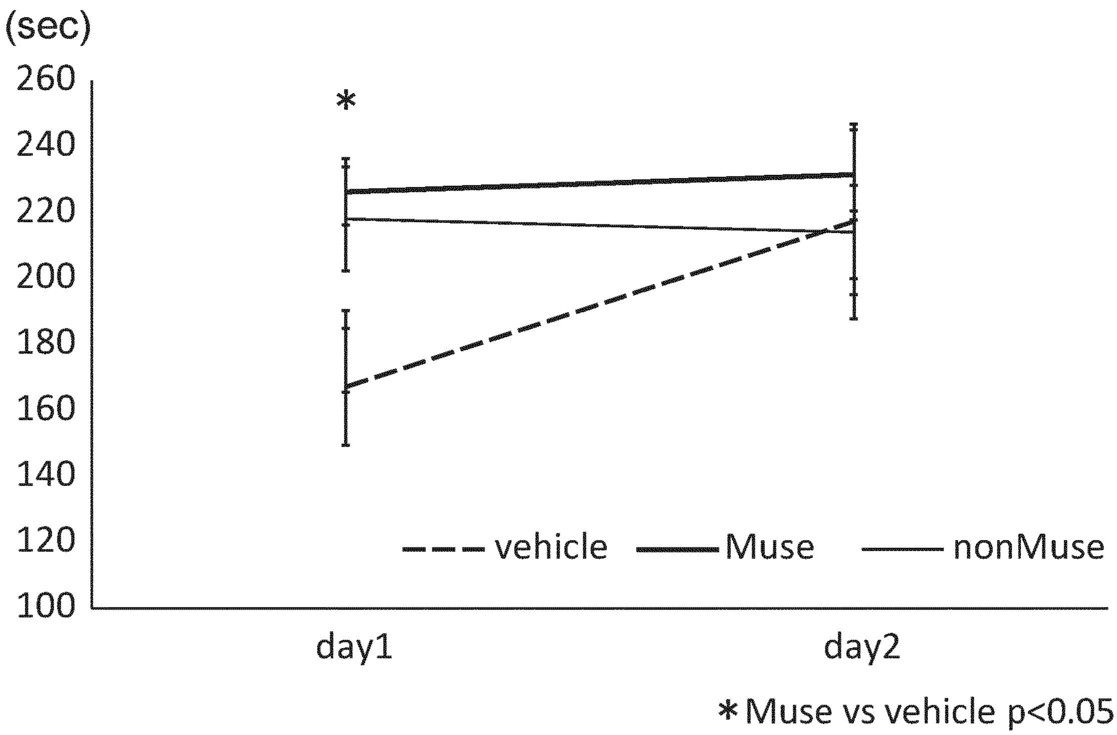
(sec)
260
240
220
200
180
160
140
120
100
*
vehicle
Muse
nonMuse
day1
day2
* Muse vs vehicle p<0.05

FIG. 3

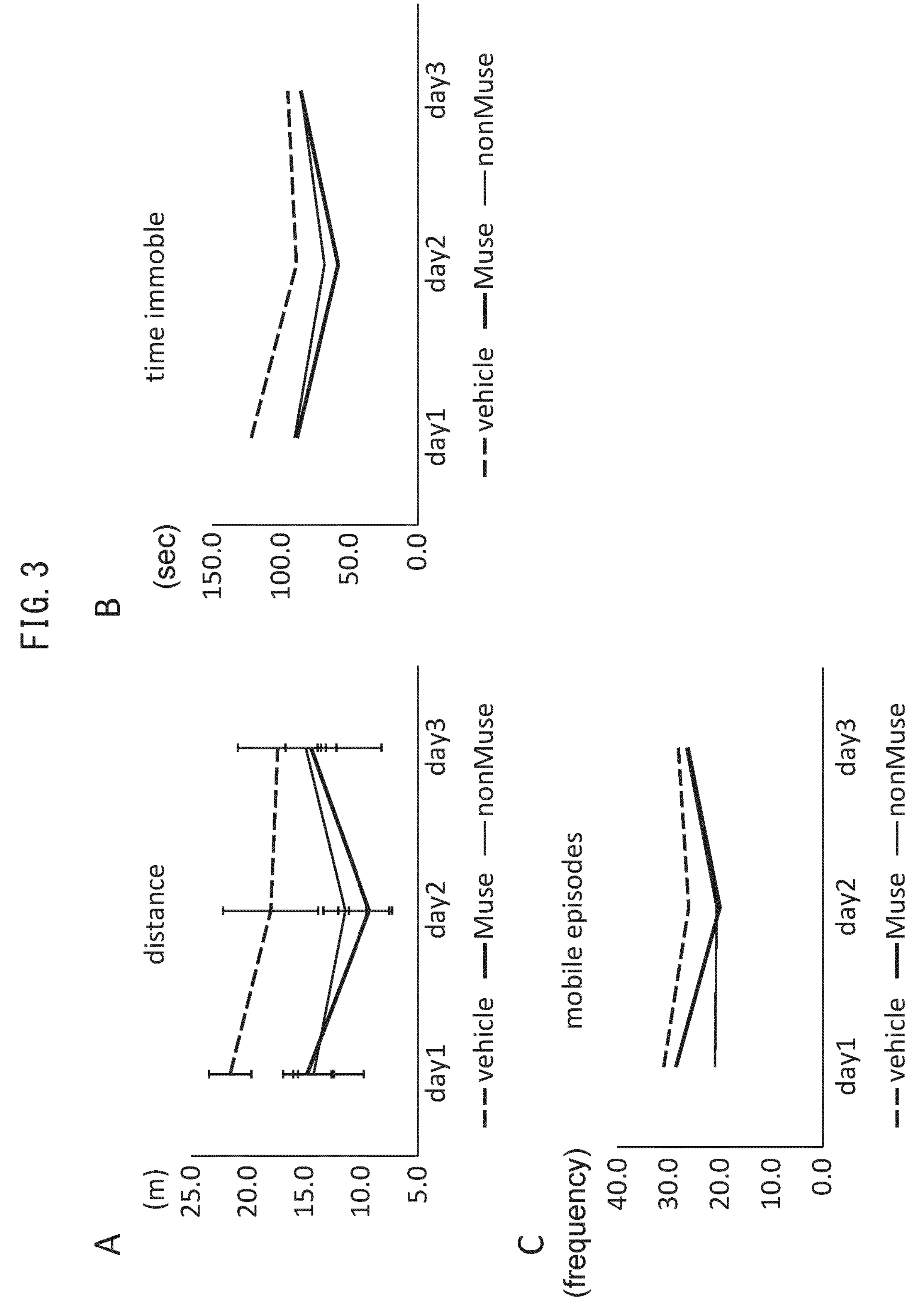
A
(m)
25.0
20.0
15.0
10.0
5.0
distance
day1
day2
day3
vehicle
Muse
nonMuse
B
(sec)
150.0
100.0
50.0
0.0
time immoble
day1
day2
day3
vehicle
Muse
nonMuse
C
(frequency)
40.0
30.0
20.0
10.0
0.0
mobile episodes
day1
day2
day3
vehicle
Muse
nonMuse

FIG. 4A

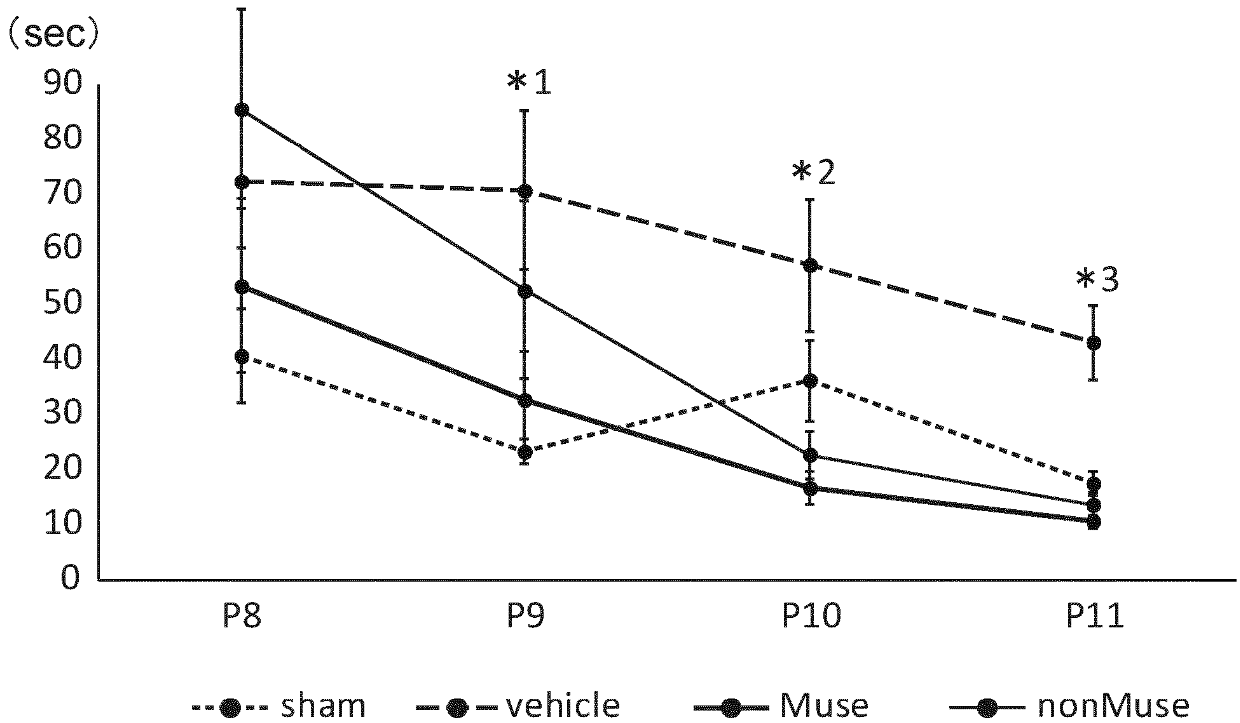


＊1 Muse vs vehicle $p<0.05$, sham vs vehicle $p<0.01$

＊2 Muse vs vehicle $p<0.01$

＊3 Muse vs vehicle $p<0.0001$, nonMuse vs vehicle $p<0.001$, sham vs vehicle $p<0.01$

FIG. 4B

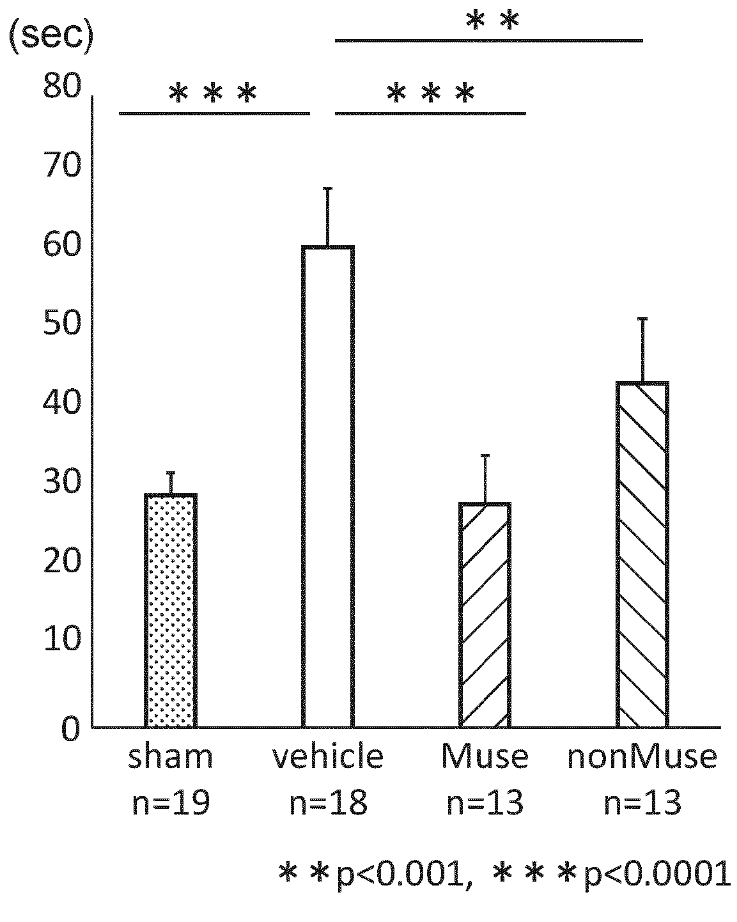
(sec)
80
70
60
50
40
30
20
10
0
＊＊＊
＊＊＊
＊＊
sham
n=19
vehicle
n=18
Muse
n=13
nonMuse
n=13
＊＊p<0.001, ＊＊＊p<0.0001

FIG. 5

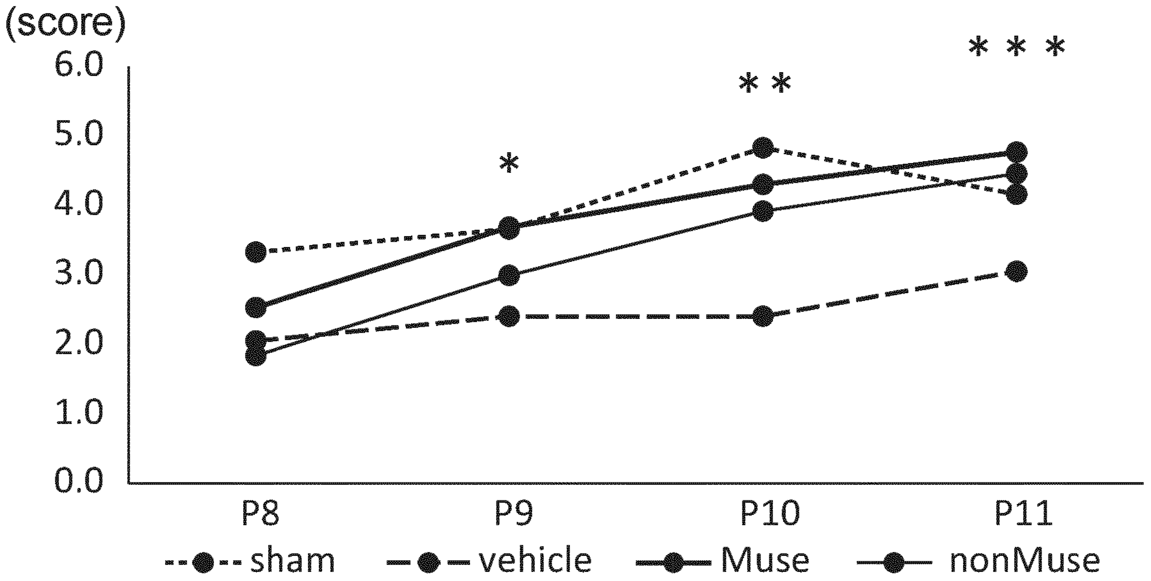


* Muse vs vehicle $p<0.05$

* * Muse vs vehicle $p<0.001$ nonMuse vs vehicle $p<0.01$ sham vs vehicle $p<0.001$

* * * Muse vs vehicle $p<0.0001$ nonMuse vs vehicle $p<0.001$ sham vs vehicle $p<0.05$

FIG. 6

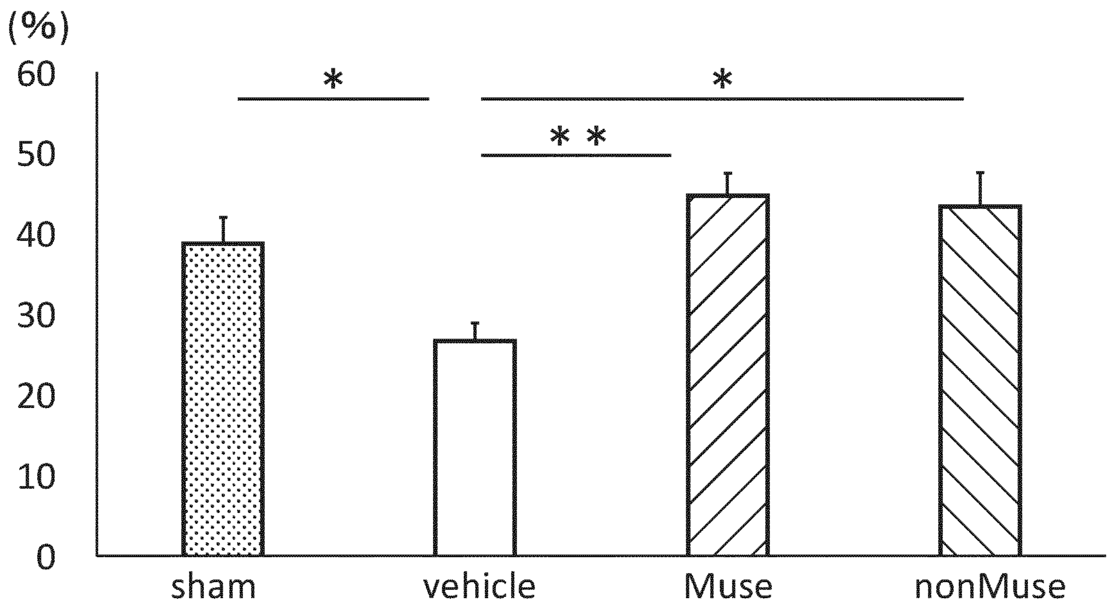


* * Muse vs vehicle $p<0.01$,

* non Muse vs vehicle $p<0.05$, sham vs vehicle $p<0.05$

FIG. 7

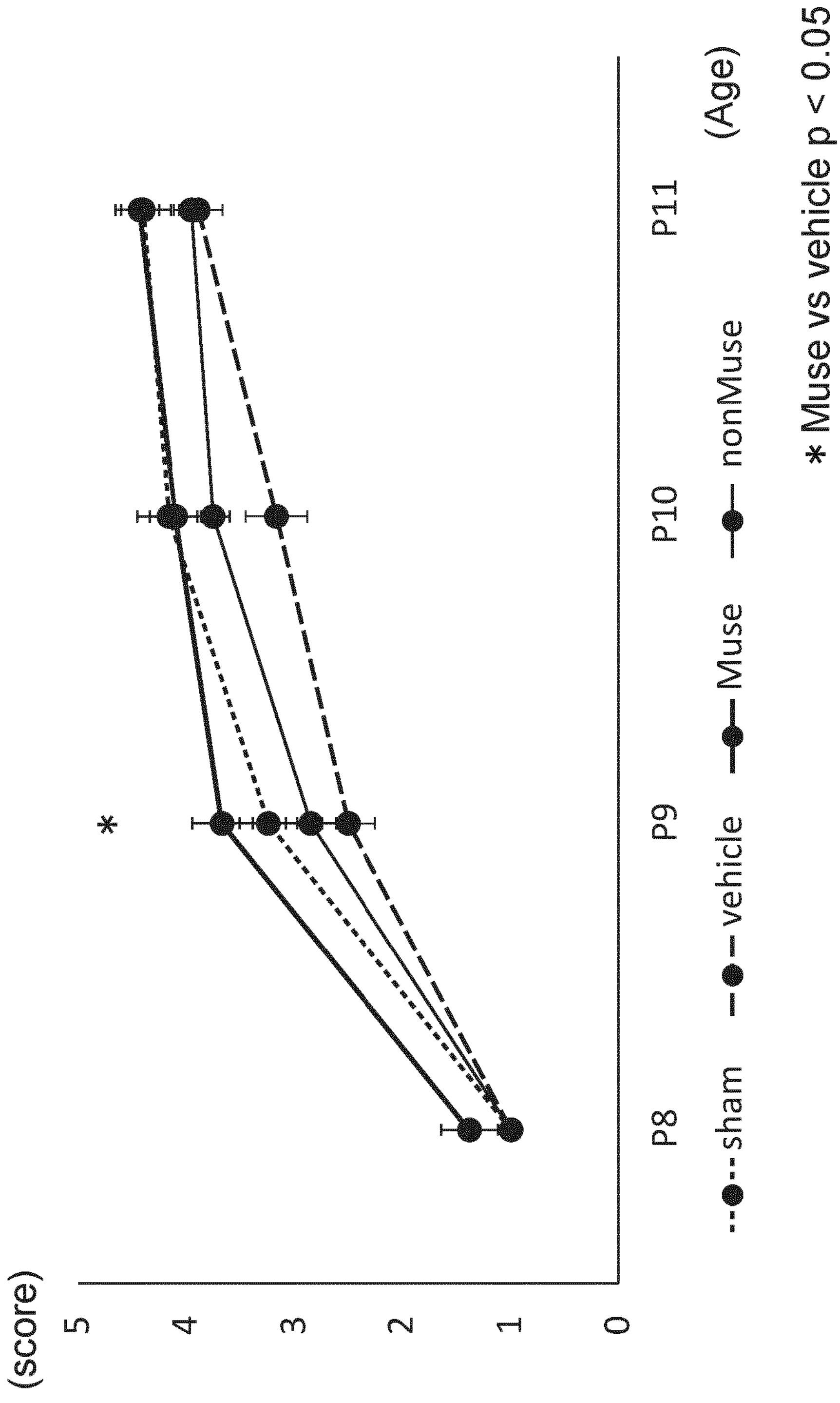
(score)
5
4
3
2
1
0
*
P8
P9
P10
P11
(Age)
sham
vehicle
Muse
nonMuse
* Muse vs vehicle p < 0.05

FIG. 8

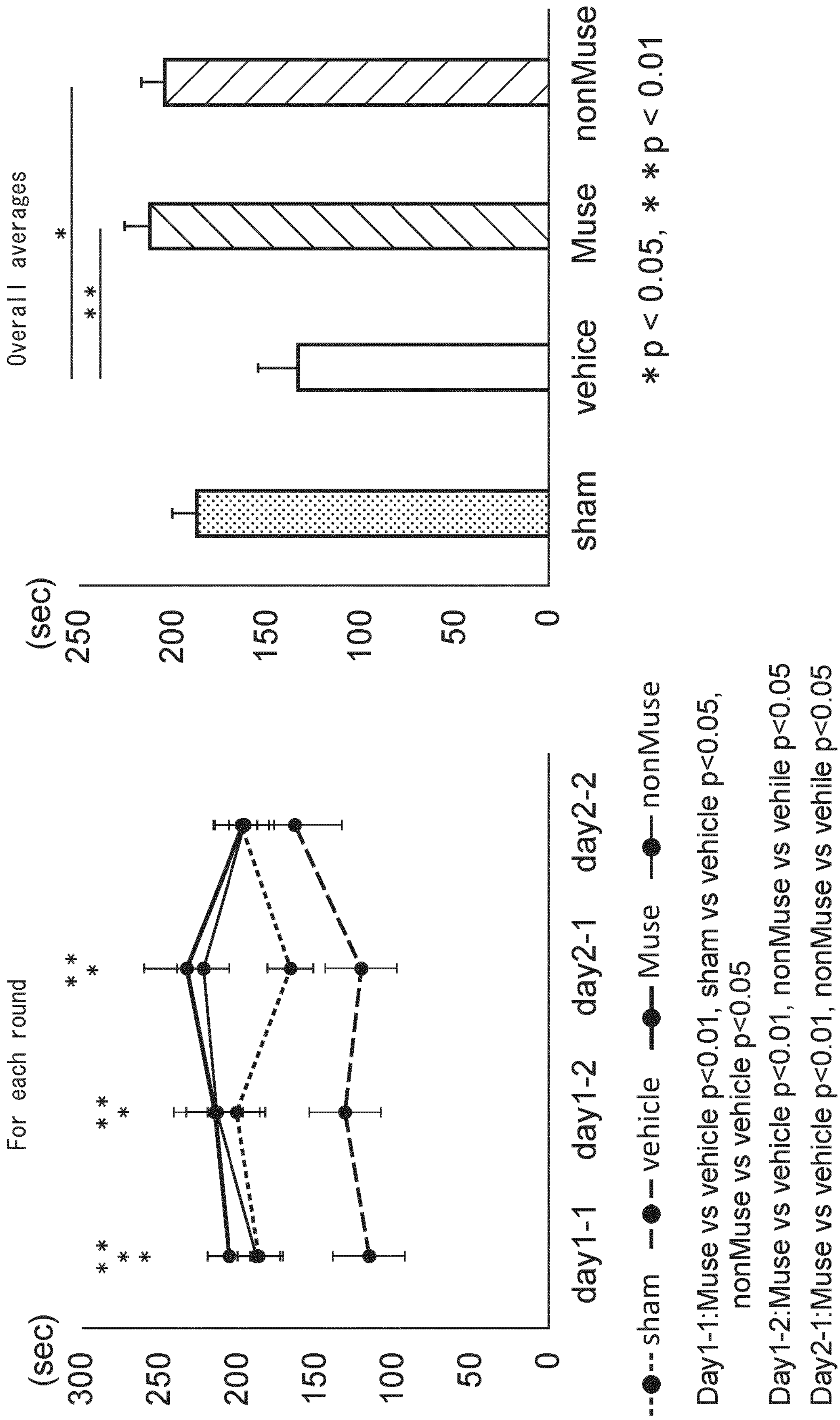
For each round
(sec)
300
250
200
150
100
50
0
**
**
**
*
*
*
day1-1
day1-2
day2-1
day2-2
sham
vehicle
Muse
nonMuse
Day1-1:Muse vs vehicle p<0.01, sham vs vehicle p<0.05, nonMuse vs vehicle p<0.05
Day1-2:Muse vs vehicle p<0.01, nonMuse vs vehile p<0.05
Day2-1:Muse vs vehicle p<0.01, nonMuse vs vehile p<0.05
Overall averages
(sec)
250
200
150
100
50
0
**
*
sham
vehice
Muse
nonMuse
* p < 0.05, ** p < 0.01

FIG. 9

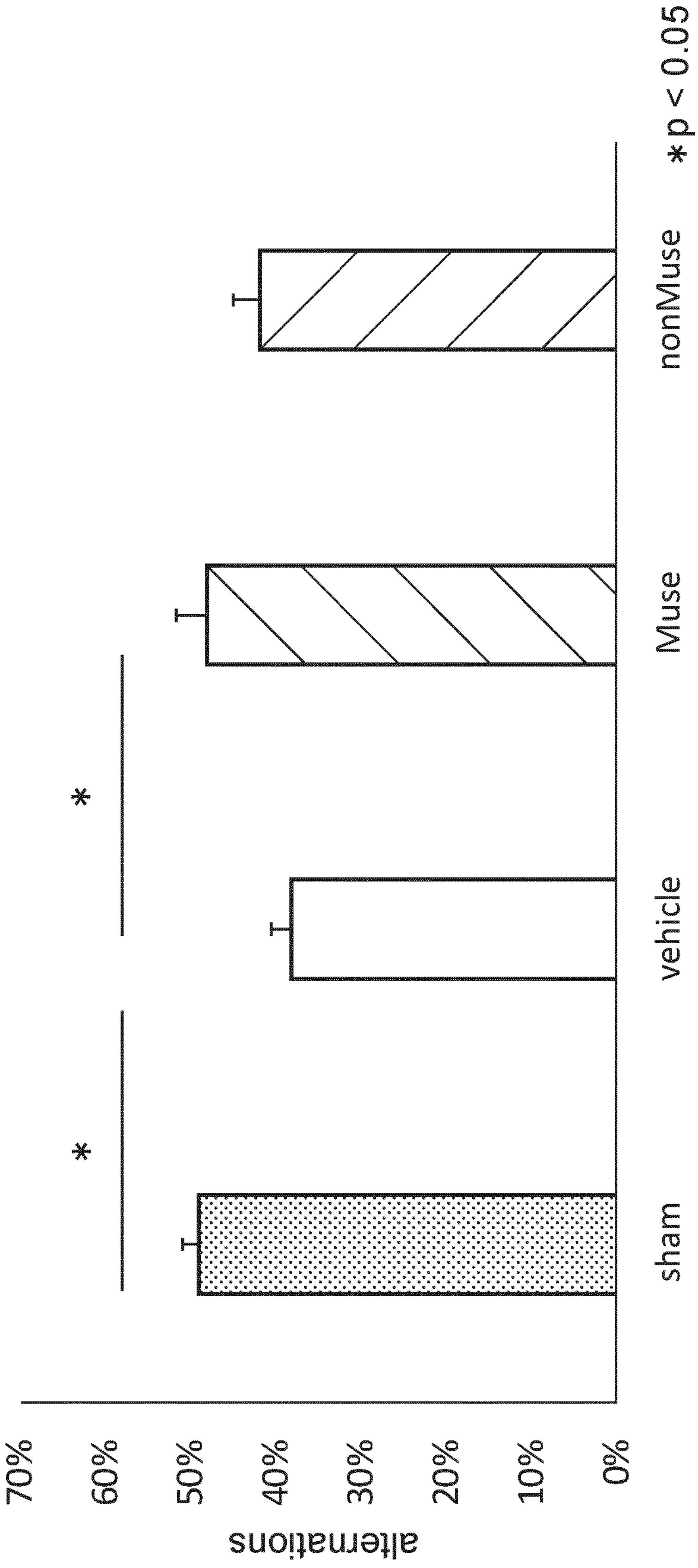
alternations
70%
60%
50%
40%
30%
20%
10%
0%
sham
vehicle
Muse
nonMuse
*
*
* p < 0.05

FIG. 10

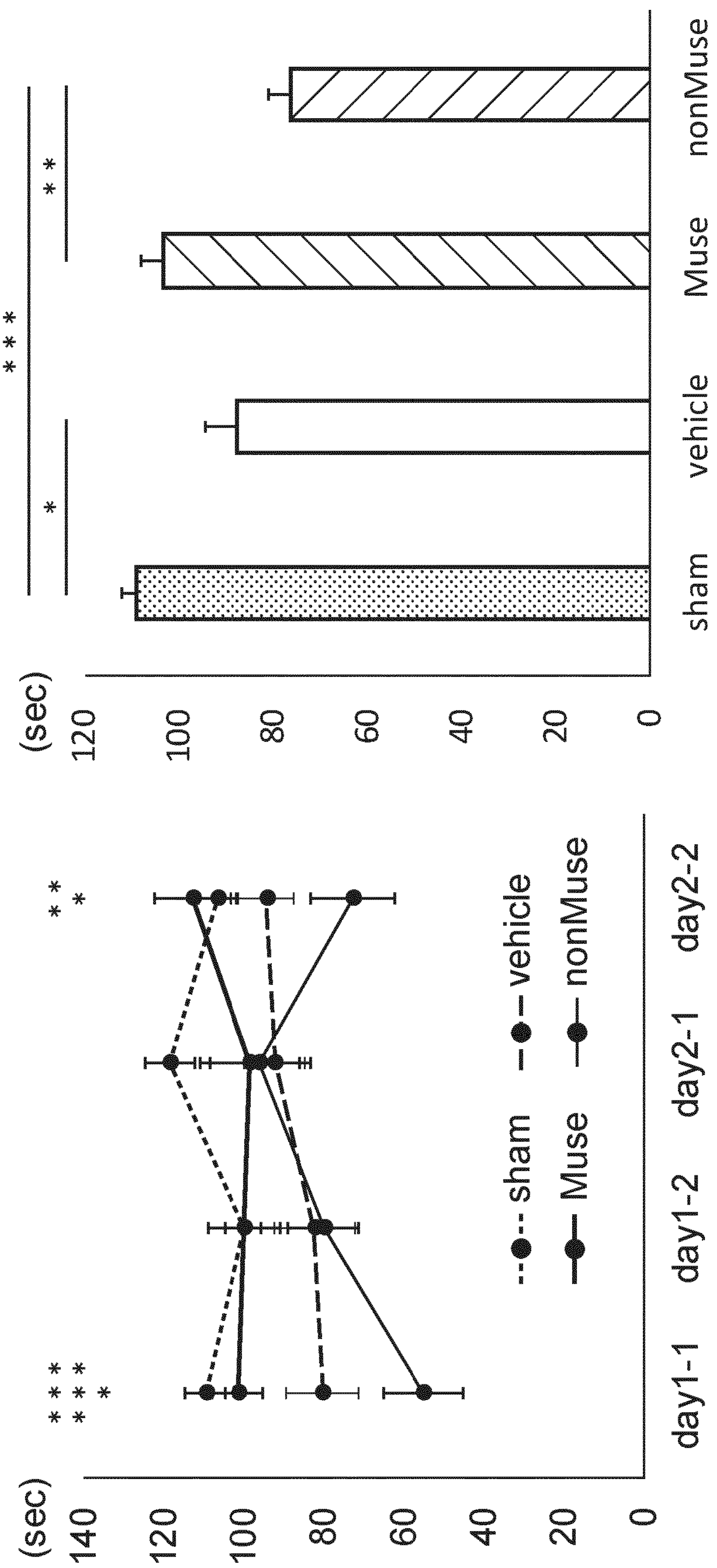


Day1-1:Muse vs nonMuse p<0.001, sham vs nonMuse p<0.001, sham vs vehicle p<0.05

Day2-2:Muse vs nonMuse p<0.01, sham vs nonMuse p<0.05

* p < 0.05, * * p < 0.01, * * * p < 0.001

FIG. 11

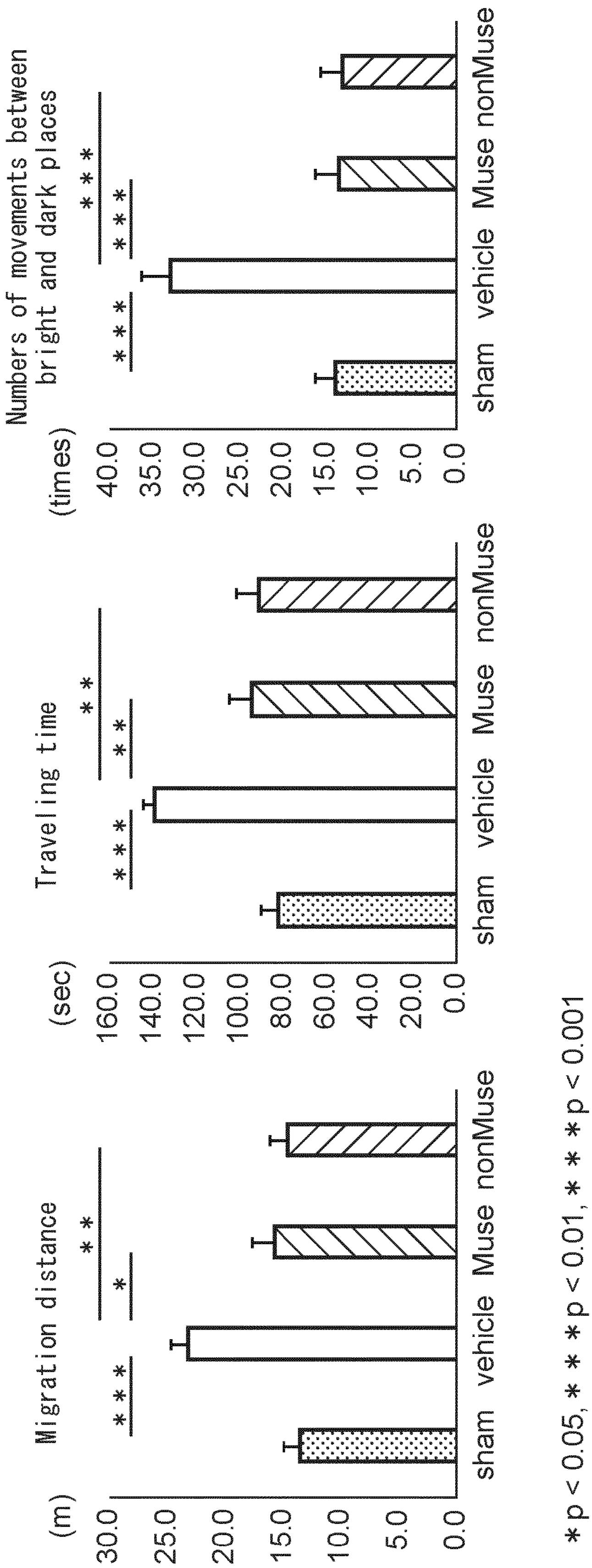
Migration distance
(m)
30.0
25.0
20.0
15.0
10.0
5.0
0.0
sham
vehicle
Muse
nonMuse
***
*
**
Traveling time
(sec)
160.0
140.0
120.0
100.0
80.0
60.0
40.0
20.0
0.0
***
**
**
Numbers of movements between bright and dark places
(times)
40.0
35.0
30.0
25.0
20.0
15.0
10.0
5.0
0.0
***
***
***
*p < 0.05, **p < 0.01, ***p < 0.001

FIG. 12

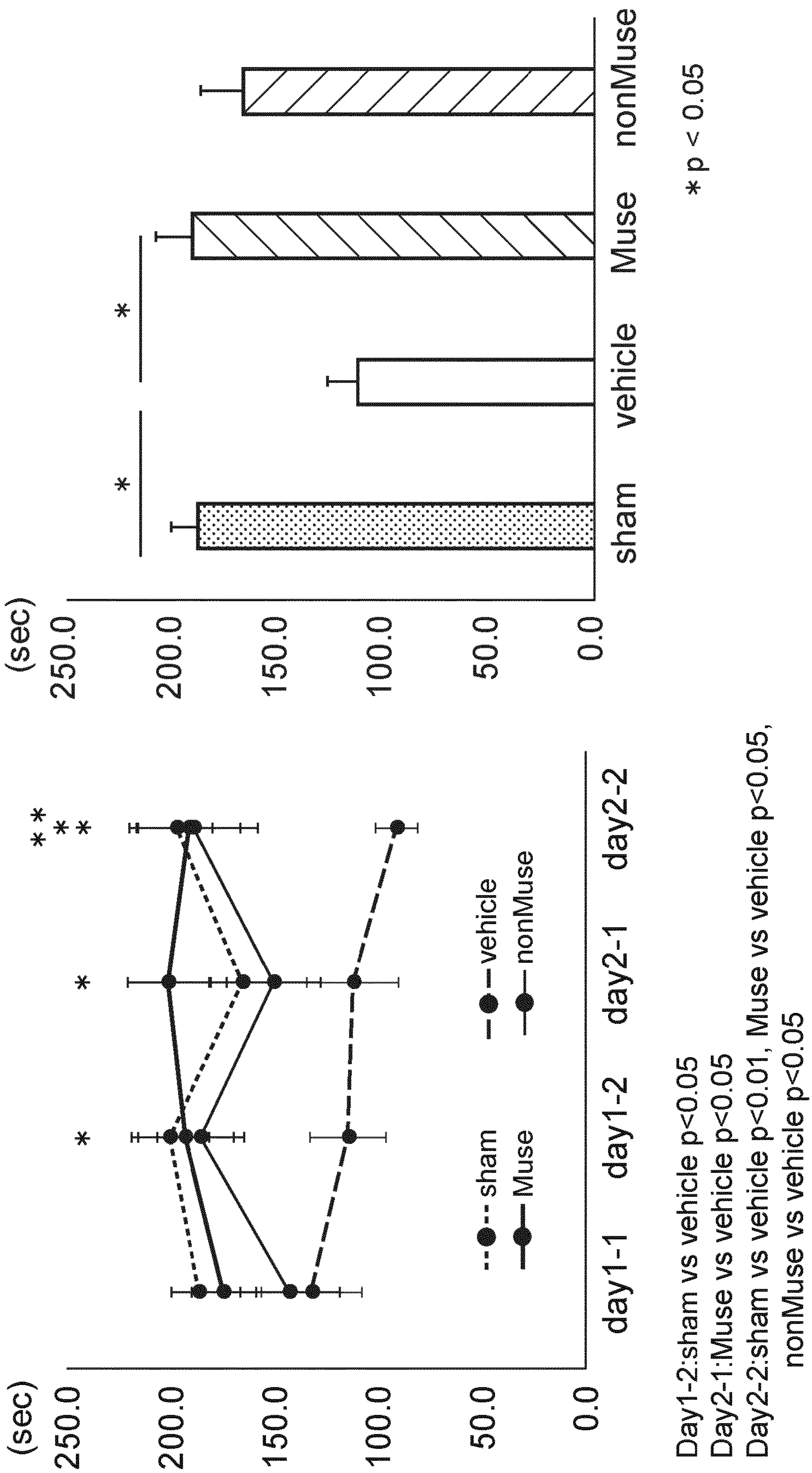
(sec)
250.0
200.0
150.0
100.0
50.0
0.0
day1-1
day1-2
day2-1
day2-2
sham
Muse
vehicle
nonMuse
*
*
* *
* *
Day1-2:sham vs vehicle p<0.05
Day2-1:Muse vs vehicle p<0.05
Day2-2:sham vs vehicle p<0.01, Muse vs vehicle p<0.05,
nonMuse vs vehicle p<0.05
(sec)
250.0
200.0
150.0
100.0
50.0
0.0
*
*
sham
vehicle
Muse
nonMuse
* p < 0.05

FIG. 13

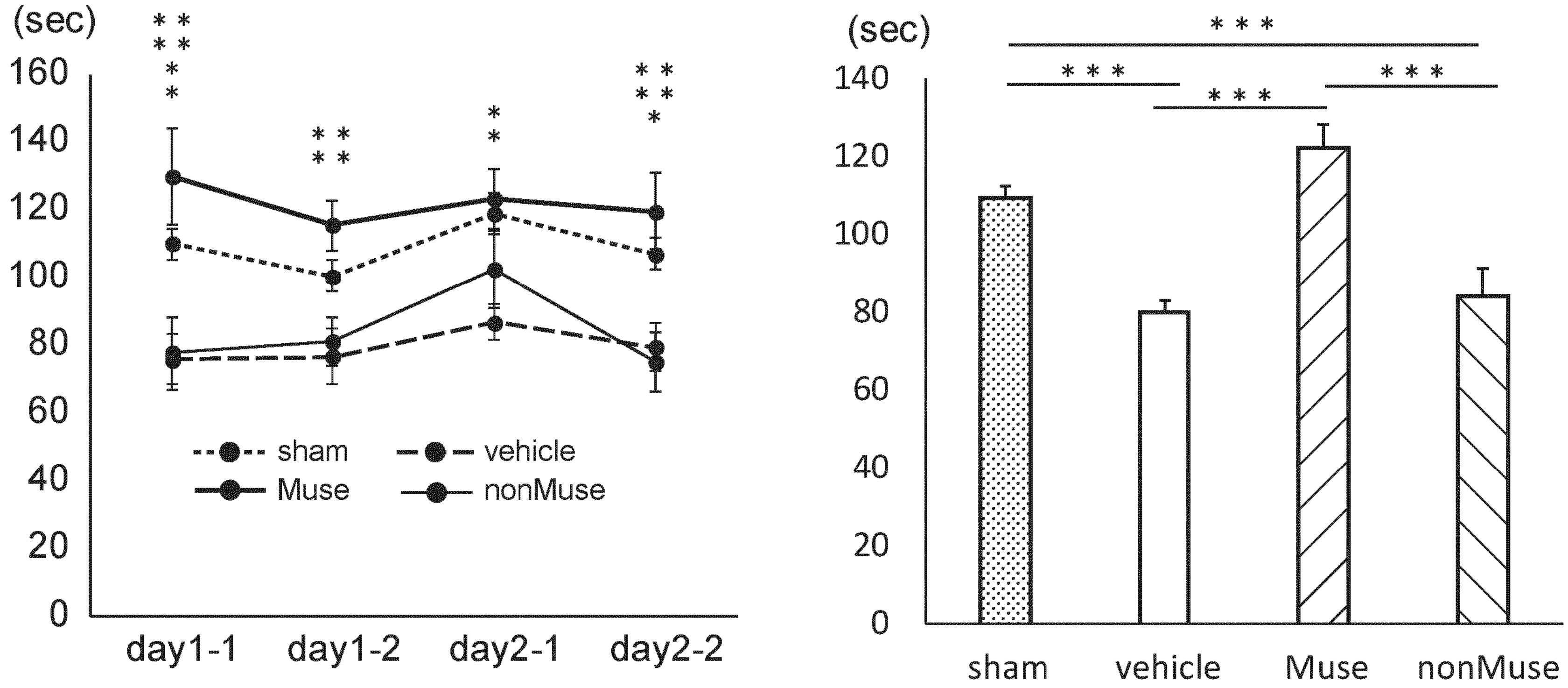


day1-1 ∗∗Muse vs nonMuse p < 0.01,∗∗Muse vs vehicle p < 0.01
∗sham vs non Muse p < 0.05,∗sham vs vehicle p < 0.05
day1-2 ∗∗Muse vs nonMuse p < 0.01,∗∗Muse vs vehicle p < 0.01
day2-1 ∗Muse vs vehicle p < 0.05,∗∗sham vs vehicle p < 0.05
day2-2 ∗∗Muse vs nonMuse p < 0.01,∗∗Muse vs vehicle p < 0.01
∗sham vs non Muse p < 0.05

∗∗∗p < 0.001

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4183742 B
- WO 2011007900 A
- WO 2017199976 A


### Non-patent literature cited in the description


- **O'KEEFFE M.J. et al.** *Pediatrics*, 2003, vol. 112, 301-307
- **DEZAWA, M. et al.** *J. Clin. Invest.*, 2004, vol. 113, 1701-1710
- **DEZAWA, M. et al.** *Science*, 2005, vol. 309, 314-317
- **WAKAO, S et al.** *Proc. Natl. Acad. Sci. USA*, 2011, vol. 108, 9875-9880
- **OGURA, F. et al.** *Stem Cells Dev.*, 20 November 2013